(19) Europäisches Patentamt / European Patent Office / Office européen des brevets

(11) **EP 3 839 481 B1**

(12) **EUROPÄISCHE PATENTSCHRIFT**

(45) Veröffentlichungstag und Bekanntmachung des Hinweises auf die Patenterteilung:
**26.02.2025   Patentblatt 2025/09**

(21) Anmeldenummer: **19218978.5**

(22) Anmeldetag: **20.12.2019**

(51) Internationale Patentklassifikation (IPC):
*G01N 21/25* (2006.01)    *G01N 21/3577* (2014.01)
*G01N 21/359* (2014.01)    *G01N 21/64* (2006.01)
*G01N 35/02* (2006.01)    *B01L 3/00* (2006.01)
*G01F 23/292* (2006.01)    *C12M 1/32* (2006.01)
*C12M 1/00* (2006.01)    *G01N 21/01* (2006.01)

(52) Gemeinsame Patentklassifikation (CPC):
*G01N 21/253; B01L 3/50853; C12M 23/12;*
*C12M 23/38; G01F 23/292; G01N 21/3577;*
*G01N 21/359; G01N 21/6452; G01N 35/028;*
*B01L 2200/142; B01L 2300/0829; G01N 2021/0143;*
*G01N 2021/6484*

(54) **VERFAHREN ZUM REDUZIEREN EINER FLÜSSIGKEITS-VERDUNSTUNG AUS WELLS EINER MIKROPLATTE**

METHOD FOR REDUCING LIQUID EVAPORATION FROM WELLS OF A MICROPLATE

PROCÉDÉ DE RÉDUCTION DE L'ÉVAPORATION DE LIQUIDE À PARTIR DE PUITS D'UNE MICROPLAQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**23.06.2021   Patentblatt 2021/25**

(73) Patentinhaber: **TECAN TRADING AG**
**8708 Männedorf (CH)**

(72) Erfinder:
• **Lansing, Manfred**
**5020 Salzburg (AT)**
• **Eggenhofer, Nicole**
**5431 Kuchl (AT)**
• **Sawetzki, Tobias**
**83483 Bischofswiesen (DE)**

(74) Vertreter: **Troesch Scheidegger Werner AG**
**Schwäntenmos 14**
**8126 Zumikon (CH)**

(56) Entgegenhaltungen:
EP-A1- 3 270 120        EP-B1- 2 943 797
WO-A2-2016/203320        CH-A1- 706 811
US-A- 4 800 164        US-A1- 2014 113 360
US-A1- 2016 003 859

**Beschreibung**

Die Erfindung betrifft ein Verfahren zum Reduzieren einer Flüssigkeits-Verdunstung aus Wells einer Mikroplatte und einen Mikroplatten-Reader.

Mikroplatten-Reader, mit denen auf optischem Wege die Inhalte von einem oder mehreren Wells einer Mikroplatte untersucht bzw. analysiert werden können, sind seit langem bekannt. Als Mikroplatte werden im Zusammenhang mit der vorliegenden Erfindung alle Multiwellplatten bezeichnet, die eine Vielzahl an Wells oder Behältern aufweisen, welche z.B. in einem Array angeordnet sind. Speziell bevorzugte Mikroplatten weisen zumindest annähernd die Masse und die Standfläche einer Mikroplatte nach dem SBS Standard auf, wie dieser vom American National Standards Institute (ANSI) veröffentlicht wurde. Bekannt sind beispielsweise solche Standard-Mikroplatten, deren Wells mit einem Rundboden, Flachboden oder V-Boden ausgestattet sind. Allen diesen Standard-Mikroplatten mit den unterschiedlichsten Wellformen ist gemeinsam, dass der Achsabstand der jeweils in einem Array angeordneten Wells ebenfalls normiert ist (vgl. ANSI_SBS 1-2-3-4-2004 Standard für Mikroplatten-Dimensionen aus dem Jahr 2006). Dieser Achsabstand beträgt z.B. bei 24-Well (4 × 6) Platten 18 mm, bei 96-Well (8 × 12) Platten 9 mm, bei 384-Well (16 × 24) Platten 4.5 mm und bei 1536-Well (32 × 48) Platten 2.25 mm. Die Höhe einer Standard-Mikroplatte kann je nach Typ sehr stark variieren und beträgt typischerweise zwischen 10.4 mm (z.B. 1536 V-Boden Deep Well Platte) und 44 mm (z.B. 96 Well Masterblock® von Greiner).

Bekannte Mikroplatten-Reader sind mit entsprechenden Lichtquellen und/oder Detektoren ausgerüstet, um Samples oder mit Testlösung versehene Samples, auch Proben genannt, in den Wells von Mikroplatten anhand z.B. ihrer Absorption, Fluoreszenz und/oder Lumineszenz zu untersuchen. Beispielsweise befinden sich die Samples in einer Testlösung, die den Einflüssen der Umwelt ausgesetzt ist. Insbesondere bei lange andauernden Versuchsreihen mit Zellkulturen in den Wells, die typischerweise in für sich stehenden Mikroplatten-Readern während Stunden oder gar Tagen und womöglich noch bei gegenüber der Raumtemperatur erhöhten Temperaturen durchgeführt werden, können sich Verdunstungsprobleme für die Samples oder die Samples enthaltende Testlösung einstellen. Das Verdunsten der Testlösung führt zu einer Eindickung und damit zu einer Änderung der Konzentration von Puffersubstanzen und zu untersuchenden Molekülen (Analyten). Dadurch werden beispielsweise die Wachstumsbedingungen für Zell-basierte Versuche und/oder die Reaktion von Zellen auf versuchsbedingte Einflüsse verändert. Es wurde zudem beobachtet, dass die Testlösung von in den Ecken einer Standard-Mikroplatte angeordneten Wells mehr derartigen Verdunstungsproblemen ausgesetzt ist als von in der Mitte einer Mikroplatte angeordneten Wells. Dies wiederum bedeutet, dass die Eindickung nicht homogen über alle Wells einer Mikroplatte verteilt eintritt, sondern zu Unterschieden, und damit zu nicht vergleichbaren Ergebnissen, innerhalb der gleichen Versuchsreihe, führt.

Vorrichtungen zum Verhindern oder zum Reduzieren derartiger Verdunstungsprobleme sind aus dem Stand der Technik bekannt. So offenbart das Patent EP 2943797 B1 eine Inkubationskassette zum Reduzieren einer Flüssigkeits-Verdunstung aus Wells einer Mikroplatte, wobei die Inkubationskassette einen Rahmen zum Aufnehmen einer Mikroplatte umfasst. Ferner stellt die Inkubationskassette ein mit Flüssigkeit befüllbares Reservoir in der Form eines Flüssigkeitskanals bereit, der die eingelegte Mikroplatte umgibt. Hierdurch wird die Atmosphäre in unmittelbarer Umgebung der Wells der Mikroplatte entsprechend angereichert, sodass eine Eindickung der Probenflüssigkeit durch Verdunstung verzögert werden kann.

US 4,800,164 A beschreibt ein sehr kompaktes automatisches Gerät zum Analysieren und Klonen von Zellkulturen sowie für die bakteriologische Analyse, welches es ermöglicht die Phasen der Erhaltung, des Experimentierens sowie der optischen Analyse durchzuführen. Das Gerät weist eine kleine Kammer auf, in welcher sich eine Platte mit mehreren Wells aus einem transparenten Material, eine optische Beobachtungseinheit, eine Vorrichtung zum Ansaugen/Injizieren des Zellmediums oder der Zellen und eine Motoreinheit zum Antreiben der Platte befinden. Das kleine Volumen dieser Kammer sorgt für eine automatische und konstante Befeuchtung durch Verdunstung des in den Wells enthaltenen Zellmediums. Dabei arbeitet die Injektionsvorrichtung mit einer Vorrichtung zur Kontrolle des Füllstands des in jedem Well eingespritzten flüssigen Mediums zusammen.

Im Stand der Technik kann allerdings die Flüssigkeit im Flüssigkeitskanal vollständig verdunsten, wodurch die Atmosphäre in unmittelbarer Umgebung der Mikroplattenwells nicht mehr angereichert wird. Daher müssen häufig der Flüssigkeitspegel bzw. Flüssigkeitsanteil im Flüssigkeitskanal überprüft und Flüssigkeit manuell nachgefüllt werden, um die Funktionalität aufrechtzuerhalten. Das Überprüfen und manuelle Nachfüllen des Flüssigkeitskanals sind mühsam und zeitaufwendig. Ferner nachteilig kann der Flüssigkeitskanal bei einigen Messungen nicht mit Flüssigkeit nachgefüllt werden, insbesondere bei Langzeitmessungen, ohne Störungen in das Experiment einzubringen.

Es ist daher Aufgabe der vorliegenden Erfindung ein Verfahren zum Reduzieren einer Flüssigkeits-Verdunstung aus Wells einer Mikroplatte und einen Mikroplatten-Reader vorzuschlagen, bei welchen aus dem Stand der Technik bekannte Nachteile eliminiert werden.

Diese Aufgabe wird durch ein Verfahren zum Reduzieren einer Flüssigkeits-Verdunstung aus Wells einer Mikroplatte gemäss Anspruch 1 gelöst. Ferner wird die Aufgabe durch einen Mikroplatten-Reader gemäss Anspruch 7 gelöst.

Das erfindungsgemässe Verfahren zum Reduzieren einer Flüssigkeits-Verdunstung aus Wells einer Mikroplatte umfasst:

a) Bereitstellen der Mikroplatte, mit einer Vielzahl von Wells
b) Zugeben eines Samples in wenigstens eines der Wells der Mikroplatte,
c) Einfahren der Mikroplatte oder einer mit der Mikroplatte bestückten Inkubationskassette in einen Mikroplatten-Reader,
d) Injizieren einer Flüssigkeit in ein Flüssigkeitsreservoir, welches in der Mikroplatte und/oder der Inkubationskassette vorgesehen ist,
e) Durchführen von Messungen auf die Samples in den jeweiligen Wells,
f) optisches Messen eines Flüssigkeitspegels im Flüssigkeitsreservoir der Mikroplatte und/oder der Inkubationskassette durch einen im Wesentlichen transparenten Abschnitt des Flüssigkeitsreservoirs der Mikroplatte und/oder des Flüssigkeitsreservoirs der Inkubationskassette hindurch,
g) Nachinjizieren der Flüssigkeit in das Flüssigkeitsreservoir der Mikroplatte und/oder in das Flüssigkeitsreservoir der Inkubationskassette, wenn der Flüssigkeitspegel einen vorbestimmten Schwellenwert unterschreitet,
h) Wiederholen der Schritte e) bis g), bis eine vorbestimmte Anzahl von Messzyklen erreicht ist,
i) Ausfahren der Mikroplatte oder der mit der Mikroplatte bestückten Inkubationskassette aus dem Mikroplatten-Reader,

wobei das Flüssigkeitsreservoir zwischen den Wells vorgesehen ist und durch eine Wandung der Mikroplatte begrenzt ist und zur Aufnahme einer Flüssigkeit ausgebildet ist, wobei die Mikroplatte mit einem Flüssigkeitsreservoir versehen ist und wenigstens einen transparenten Abschnitt umfasst, und/oder wobei die Inkubationskassette einen Rahmen zum Aufnehmen einer Mikroplatte mit Wells umfasst, wobei der Rahmen eine von einer inneren Wand umgebene zentrale erste Öffnung umfasst, deren Masse zum Einlegen einer Mikroplatte ausgelegt sind, und der Rahmen eine im Wesentlichen parallel zur inneren
Wand verlaufende äussere Wand umfasst, die über einen Zwischenboden an der inneren Wand anschliesst, wobei das Flüssigkeitsreservoir durch die beiden Wände und den Zwischenboden als ein die erste zentrale Öffnung umgebendes Flüssigkeitsreservoir zum Aufnehmen einer Flüssigkeit gebildet ist, wobei wenigstens ein das Flüssigkeitsreservoir ausbildender Abschnitt der Inkubationskassette wenigstens abschnittsweise mit mindestens einem transparenten Abschnitt versehen ist.
Die Schritte des erfindungsgemässen Verfahrens sind in der Reihenfolge veränderbar. Beispielsweise kann die Flüssigkeit auch ausserhalb des Mikroplatten-Readers, d.h. vor dem Einfahren der Mikroplatte oder der mit der Mikroplatte bestückten Inkubationskassette in den Mikroplatten-Reader, in das Flüssigkeitsreservoir der Mikroplatte und/oder der Inkubationskassette injiziert werden.
Eine Inkubationskassette zum Reduzieren einer Flüssigkeits-Verdunstung aus Wells einer Mikroplatte umfasst einen Rahmen zum Aufnehmen einer Mikroplatte mit Wells, wobei der Rahmen eine von einer inneren Wand umgebene, zentrale erste Öffnung umfasst, deren Masse zum Einlegen einer Mikroplatte ausgelegt sind, und der Rahmen eine im Wesentlichen parallel zur inneren Wand verlaufende äussere Wand umfasst, die über einen Zwischenboden an der inneren Wand anschliesst, so dass durch die beiden Wände und den Zwischenboden ein die erste zentrale Öffnung umgebendes Flüssigkeitsreservoir zum Aufnehmen einer Flüssigkeit gebildet ist, wobei wenigstens ein das Flüssigkeitsreservoir ausbildender Abschnitt der Inkubationskassette wenigstens abschnittsweise mit mindestens einem transparenten Abschnitt versehen ist.
Ein Mikroplatten-Reader zur Durchführung des erfindungsgemässen Verfahrens weist ein Gehäuse und eine aus dem Gehäuse ausfahrbare Transportauflage auf, wobei die Transportauflage eine Auflagefläche zum Auflegen einer Mikroplatte oder einer Inkubationskassette umfasst. Ferner umfasst der Mikroplatten-Reader einen Flüssigkeit-Injektor zur automatisierten Abgabe von Flüssigkeit in das Flüssigkeitsreservoir der Mikroplatte oder der Inkubationskassette.
Weitere bevorzugte und erfinderische Merkmale ergeben sich jeweils aus den weiteren Ansprüchen.
Vorteile der Erfindung umfassen:

- Die Überwachung des Flüssigkeitspegels im Flüssigkeitsreservoir der Mikroplatte und/oder der Inkubationskassette kann unabhängig von der Zeitdauer des Experiments mit einer intervallmässigen Messung von Proben in den Wells der Mikroplatte automatisiert vorgenommen werden.
- Durch den transparenten Abschnitt der Inkubationskassette und/oder den transparenten Abschnitt der in die Inkubationskassette eingelegten Mikroplatte sind diese von unten hindurch optisch zugänglich, so dass optische Messungen zur Bestimmung des Flüssigkeitspegels im Flüssigkeitsreservoir angewendet werden können. Das Licht zur jeweiligen optischen Messung läuft durch den jeweiligen transparenten Abschnitt.
- Das Flüssigkeitsreservoir der Mikroplatte und/oder der Inkubationskassette kann manuell oder durch einen Injektor automatisiert mit Flüssigkeit aufgefüllt bzw. nachgefüllt werden, sobald erfasst wird, dass der Flüssigkeitspegel im Flüssigkeitsreservoir einen vordefinierten Flüssigkeitspegel unterschreitet. Hierdurch bleibt das Flüssigkeitsreservoir zuverlässig mit Flüssigkeit gefüllt.

- Das Nachinjizieren der Flüssigkeit in das Flüssigkeitsreservoir der Mikroplatte und/oder der Inkubationskassette kann durch einen bereits im Mikroplatten-Reader umfassten Injektor durchgeführt werden. Somit sind die Samples bzw. die mit Testlösung versehenen Samples vorteilhaft reduzierten Schwankungen der Temperatur und/oder Atmosphäre ausgesetzt.
- Zur automatisierten Überwachung des Flüssigkeitspegels kann auf zumindest eine Einrichtung zur optischen Messung zurückgegriffen werden, insbesondere eine Einrichtung zur Absorptionsmessung.
- Der transparente Abschnitt der Mikroplatte oder Inkubationskassette ermöglicht einen Lichteinfall von unterhalb auf die Flüssigkeit zur optischen Messung des Flüssigkeitspegels mittels z.B. Absorptionsmessung.
- Die entsprechende Einrichtung zur optischen Messung des Flüssigkeitspegels im Flüssigkeitsreservoir der Mikroplatte und/oder der Inkubationskassette kann bereits im Mikroplatten-Reader umfasst sein, wie z.B. eine Einrichtung zum optischen Analysieren von Proben in den Wells der Mikroplatte, insbesondere zur Absorptionsmessung. Hierdurch werden Kosten und Platz eingespart, da auf eine bereits im Mikroplatten-Reader umfasste Einrichtung zurückgegriffen werden kann.
- Mittels Bestimmung der Lichtabsorption kann der Flüssigkeitspegel der Flüssigkeit im Flüssigkeitsreservoir der Mikroplatte oder Inkubationskassette präzise bestimmt werden. Im Allgemeinen lassen sich durch den durch die Flüssigkeit absorbierten Lichtanteil Rückschlüsse ziehen auf die Konzentration der Flüssigkeit bzw. auf den Flüssigkeitspegel.
- Die Inkubationskassette ist so dimensioniert und ausgestaltet, dass diese einfach (manuell oder robotisiert) in die Transportauflage eines Mikroplatten-Readers eingesetzt und ebenso einfach (manuell oder robotisiert) wieder von dieser Transportauflage entfernt werden kann.
- Um die angereicherte Atmosphäre von der Umgebung abzuschirmen, kann ein Deckel auf die Inkubationskassette aufgelegt werden. Dieser Deckel kann innerhalb des Mikroplatten-Readers abgehoben und wieder aufgesetzt werden, so dass die Mikroplattenwells für die Zeit aller notwendigen Aktionen frei zugänglich sind. Im Falle der Verwendung einer Mikroplatte ohne Inkubationskassette kann auch ein Deckel auf die Mikroplatte aufgelegt werden.
- Der für Zellkulturen bzw. für Zell-basierte Versuche notwendige Gasaustausch zwischen den Mikroplattenwells und der Umgebung kann mittels sporadischen Abhebens des Deckels der Inkubationskassette bzw. Deckels der Mikroplatte durch das Bedienungspersonal, durch einen Roboter oder durch eine entsprechende Einrichtung im Mikroplatten-Reader selbst unterstützt werden.
- In den Inkubationsrahmen bzw. in die Inkubationskassette können beliebige Mikroplatten eingelegt werden, wobei Standard-Mikroplatten gemäss ANSI_SBS 1-2-3-4-2004 Standard bevorzugt sind. Damit wird zum Durchführen von Versuchen mit Zellkulturen bzw. von Zell-basierten Versuchen das Verwenden von Spezial-Mikroplatten überflüssig.
- Vorteilhaft bedingt durch die Flüssigkeit im Reservoir der Inkubationskassette und/oder Mikroplatte und der Möglichkeit des automatisierten Nachfüllens des Reservoirs können in einem Mikroplatten-Reader Langzeitversuche selbst bei erhöhter Temperatur (z.B. bei 37°C) durchgeführt werden.

Die Erfindung wird anhand von schematischen Figuren in der Zeichnung beispielhaft gezeigt. Die Figuren sollen ausgewählte Ausführungsformen des Erfindungsgegenstandes dokumentieren, aber den Umfang der vorliegenden Erfindung nicht einschränken. Dabei zeigen:

Fig. 1     eine Ansicht einer Inkubationskassette mit abgenommenem Deckel auf einer Transportauflage eines Mikroplatten-Readers, wobei die Inkubationskassette mit transparenten Abschnitten versehen ist;

Fig. 2A,B     Detailansichten einer Mikroplatte mit unterschiedlich gefüllten Flüssigkeitsreservoirs;

Fig. 3A,B     eine Ansicht eines Rahmens einer Inkubationskassette mit eingelegter Mikroplatte in einer Ansicht von oberhalb, sowie eine Detailansicht eines transparenten Abschnitts des Rahmens der Inkubationskassette;

Fig. 4     eine perspektivische Ansicht eines Rahmens einer Inkubationskassette mit eingelegter Mikroplatte mit einem teilweise abgehobenen Deckel;

Fig. 5A,B     vertikale Teilschnitte durch eine zugedeckte Inkubationskassette in unterschiedlichen Ausführungen mit jeweils einem schematisch dargestellten transparenten Abschnitt;

Fig. 6     einen vertikalen Schnitt durch einen Mikroplatten-Reader, bei welchem eine Transportauflage mit aufgesetzter Inkubationskassette und hierin eingelegter Mikroplatte ausgefahren ist;

Fig. 7        einen vertikalen Schnitt durch einen weiteren Mikroplatten-Reader, bei welchem eine Transportauflage mit aufgesetzter Mikroplatte ausgefahren ist;

Fig. 8A,B     Kurvenverläufe einer Absorptionsmessung der Flüssigkeit im Flüssigkeitsreservoir bei unterschiedlichen Wellenlängen, jeweils aufgetragen in Abhängigkeit von der Zeit;

Fig. 9A,B     ein Ablaufdiagramm eines Verfahrens zum Reduzieren einer Flüssigkeits-Verdunstung aus Wells einer in einer Inkubationskassette eingelegten Mikroplatte; und

Fig. 10A,B    ein Ablaufdiagramm eines Verfahrens zum Reduzieren einer Flüssigkeits-Verdunstung aus Wells einer Mikroplatte.

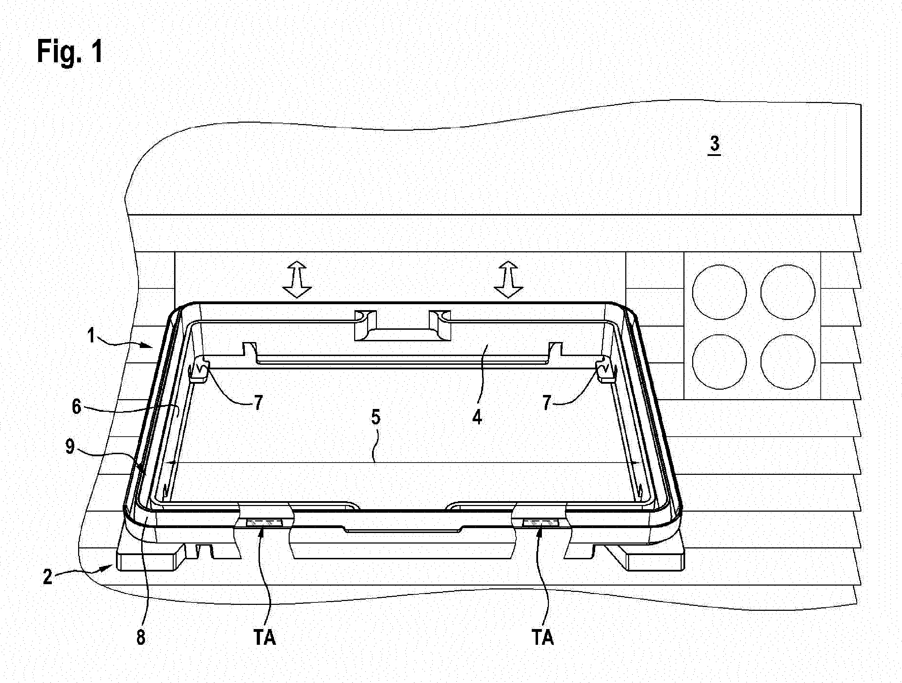

Die Figur 1 zeigt eine Ausführung von einer Inkubationskassette 1, welche auf einer Transportauflage 2 eines Mikroplatten-Readers 3 zum Transportieren der Inkubationskassette 1 aufgelegt ist. In die Inkubationskassette 1 kann wiederum eine Mikroplatte, z.B. eine Standard-Mikroplatte, eingesetzt werden (nicht gezeigt).

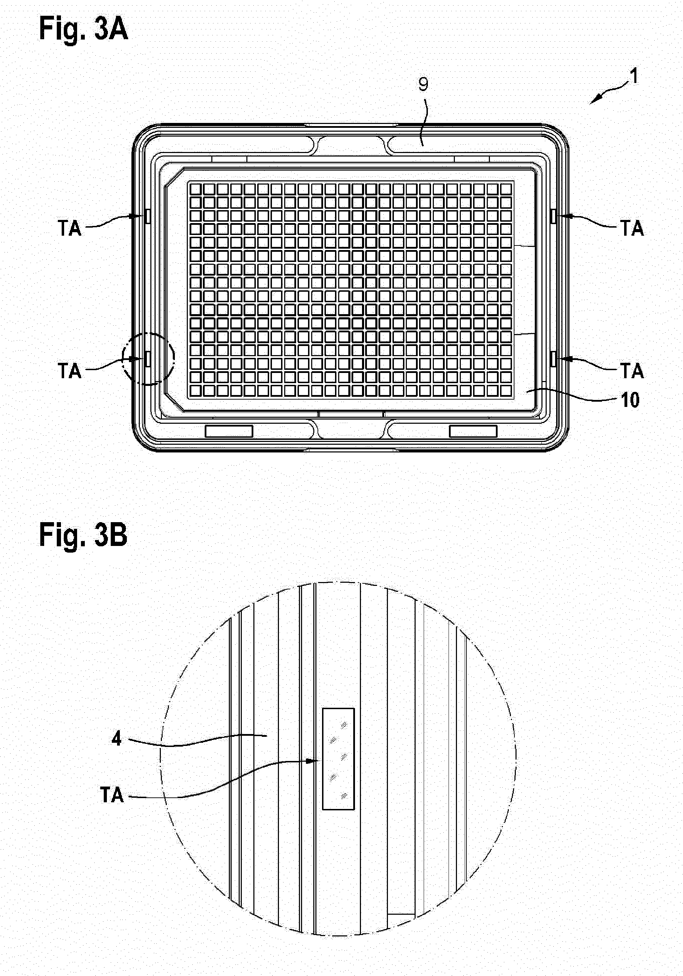

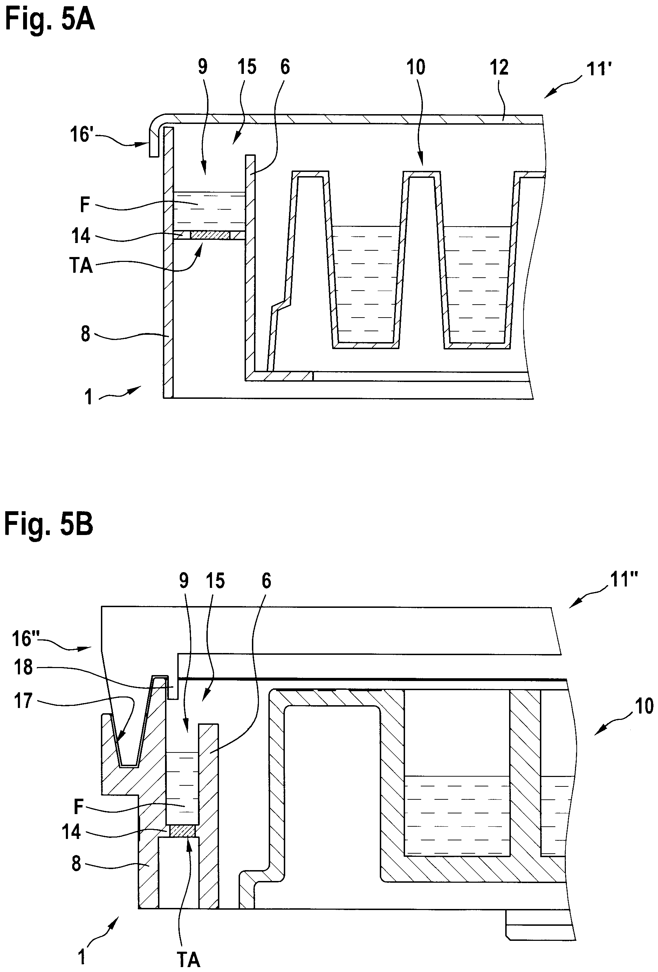

Die Inkubationskassette 1 umfasst einen Rahmen 4, auf welchen ein Deckel aufgesetzt werden kann (nicht gezeigt). Der Rahmen 4 umfasst eine zentrale erste Öffnung 5, deren Masse zum vollständigen Einlegen der Mikroplatte ausgelegt sind. Dabei ist die zentrale erste Öffnung 5 von einer vorzugsweise im Wesentlichen senkrechten inneren Wand 6 umgeben, wobei bevorzugt an deren unterem Ende zumindest abschnittsweise mehrere im Wesentlichen horizontale Tragflächen 7 angeordnet sind. Diese Tragflächen 7 dienen zum Tragen der eingelegten Mikroplatte (nicht gezeigt). Der Rahmen 4 der Inkubationskassette 1 umfasst zudem eine bevorzugt im Wesentlichen parallel zur inneren Wand 6 verlaufende äussere Wand 8, die über einen Zwischenboden an der inneren Wand 6 anschliesst, so dass durch die beiden Wände 6,8 und den Zwischenboden ein die zentrale erste Öffnung 5 umgebender Kanal, auch Flüssigkeitsreservoir 9 genannt, zum Aufnehmen einer Flüssigkeit gebildet ist (siehe auch Fig. 3A,5A,B).

Das Flüssigkeitsreservoir 9 ist abschnittsweise mit wenigstens einem transparenten Abschnitt TA versehen. Mit anderen Worten, ist wenigstens ein das Flüssigkeitsreservoir 9 ausbildender Abschnitt der Inkubationskassette 1, bevorzugt der Zwischenboden, wenigstens abschnittsweise mit mindestens einem transparenten Abschnitt TA versehen. Wie hier verwendet, umfassen Abschnitte des Flüssigkeitsreservoirs 9 die innere Wand 6, die äussere Wand 8 und/oder den Zwischenboden. Der transparente Abschnitt TA ist derart angeordnet, dass er mit der im Flüssigkeitsreservoir 9 aufgenommenen Flüssigkeit in Kontakt steht. In dem gezeigten Beispiel, und bevorzugt, ist der transparente Abschnitt TA im Zwischenboden angeordnet. Obwohl nicht gezeigt, kann der transparente Abschnitt TA in wenigstens einer der Wände 6,8 angeordnet sein. Der transparente Abschnitt TA enthält ein optisch transparentes Material, welches optisch transparent sein kann für Licht z.B. einer Messvorrichtung zur Absorptionsmessung der Flüssigkeit und somit zum Bestimmen einer optischen Weglänge bzw. eines Pegels der aufgenommenen Flüssigkeit. Somit ermöglicht der transparente Abschnitt TA, dass der Flüssigkeitsanteil bzw. Flüssigkeitspegel im Flüssigkeitsreservoir 9 mittels optischer Messung überwacht werden kann. Das jeweilige Licht zur optischen Messung durchläuft hierbei den transparenten Abschnitt TA von unterhalb der Inkubationskassette 1. Die optische Messung kann automatisiert vorgenommen werden, und zwar unabhängig von der Zeitdauer einer jeweiligen Messung bzw. Analyse von Proben in Wells einer Mikroplatte.

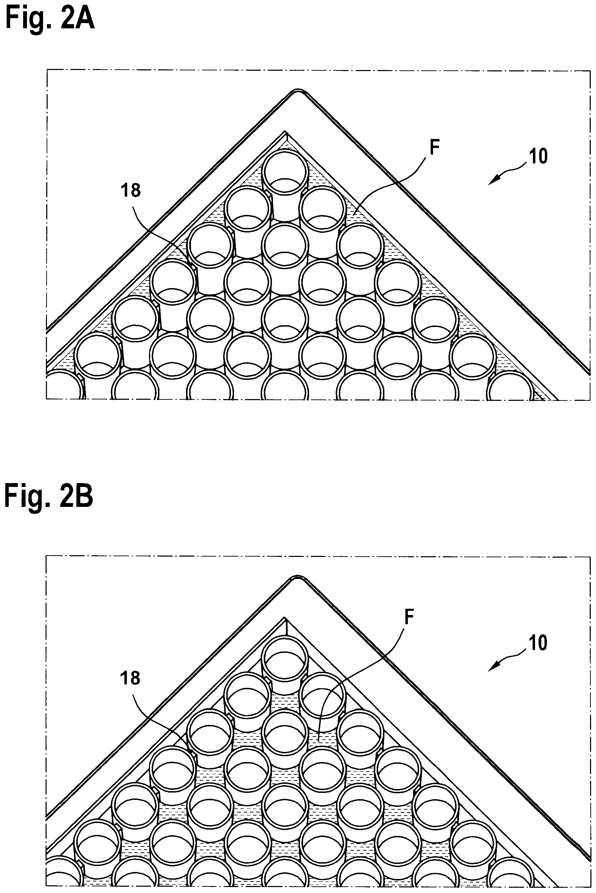

Die Figuren 2A,B zeigen Detailansichten einer Mikroplatte 10. Die Mikroplatte 10 enthält mehrere Wells, welche vom Boden der Mikroplatte 10 nach oben geöffnet herausragen. Die Mikroplatte 10 ist wiederum durch eine Wandung nach aussen abgegrenzt. Der somit gebildete Zwischenraum bildet ein Reservoir, auch als Flüssigkeitsreservoir bezeichnet, zur Aufnahme von Flüssigkeit F. Bei der in den Figuren gezeigten Mikroplatte 10 sind die jeweils parallel zur Wandung verlaufenden, äusseren Wells durch Stege 12 verbunden. Diese Stege 12 teilen das Flüssigkeitsreservoir in zwei Teilreservoire auf. Wie in den Figuren zu erkennen, befindet sich ein erstes Teilreservoir im Abschnitt der Mikroplatte 10 zwischen den Stegen 12 (und äusseren Wells) und der Wandung und ist in Fig. 2A mit Flüssigkeit F gefüllt gezeigt. Ein weiteres Teilreservoir wird durch den restlichen (inneren) Abschnitt der Mikroplatte 10 gebildet und ist in Fig. 2B mit Flüssigkeit F gefüllt gezeigt. Obwohl nicht gezeigt, können auch beide Teilreservoire mit Flüssigkeit F gefüllt sein. Die Mikroplatte 10 kann ganz oder teilweise aus transparentem Material ausgebildet sein.

Fig. 3A zeigt eine Ansicht eines Rahmens 4 einer Inkubationskassette 1 mit eingelegter Mikroplatte 10 (z.B. eine 384-Well Standard-Mikroplatte) in einer Ansicht von oben, und Fig. 3B zeigt eine Detailansicht eines transparenten Abschnitts TA des Rahmens 4 der Inkubationskassette 1. Der Rahmen 4 der Inkubationskassette 1 ist im Bereich des Flüssigkeitsreservoirs 9 (siehe auch Ansicht in Fig. 1) mit z.B. sechs transparenten Abschnitten TA versehen, durch welche hindurch (von unterhalb) optische Messungen zum Bestimmen des Flüssigkeitspegels im Flüssigkeitsreservoir 9 der Inkubationskassette 1 vorgenommen werden können. Die Mikroplatte 10 selber kann vollständig transparent sein, sodass zusätzlich oder alternativ durch diese hindurch ein Flüssigkeitspegel von einer im Flüssigkeitsreservoir der Mikroplatte 10 aufgenommenen Flüssigkeit erfasst werden kann.

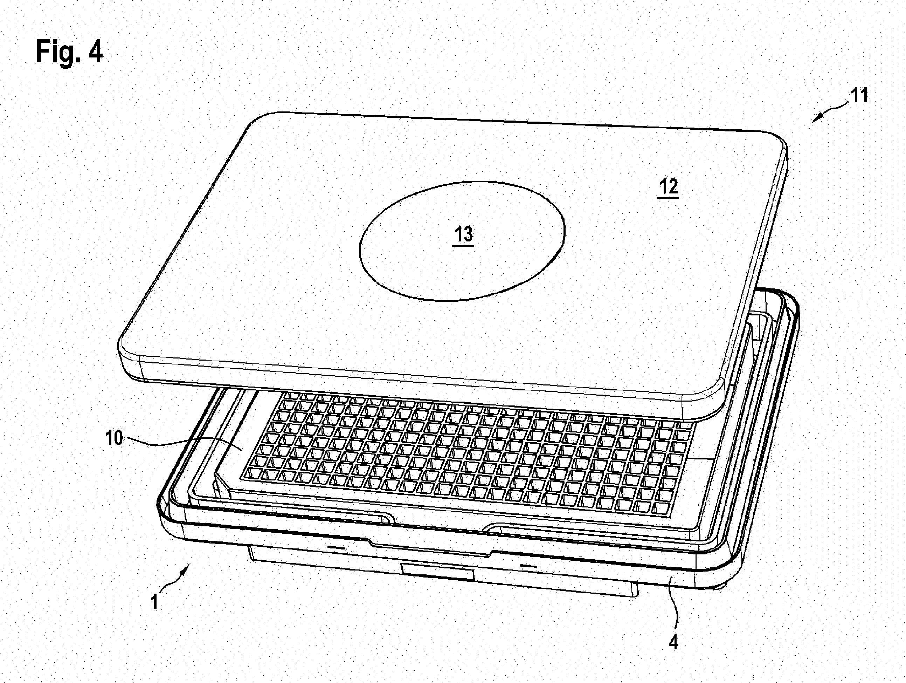

Fig. 4 zeigt eine perspektivische Ansicht eines Rahmens 4 einer Inkubationskassette 1 mit eingelegter Mikroplatte 10, mit einem teilweise abgehobenen Deckel 11. Der Deckel 11 der Inkubationskassette 1 dient zum Abdecken des Rahmens 4 mit eingelegter Mikroplatte 10. Das Auflegen oder Abheben des Deckels 11 kann innerhalb oder ausserhalb eines Mikroplatten-Readers (nicht gezeigt) manuell oder mit einem Roboter erfolgen. Der abgebildete Deckel 11 umfasst eine Platte 12 mit einer magnetisierbaren Oberfläche 13, welche lediglich einen Teil der Platte 12 bedecken kann. Alternativ könnten mehrere derartig kleine magnetisierbare Oberflächen oder eine einzige grosse magnetisierbare Oberfläche vorgesehen sein, welche zumindest annähernd die ganze Platte 12 abdeckt. Die magnetisierbare Oberfläche 13 kann ausgewählt sein aus einer Gruppe, die eine selbstklebende Metall-Folie, eine umspritzte Metall-Platte und eine angeklebte Metall-Platte umfasst, und wobei das Metall umfassen kann: Eisen, Nickel und deren Legierungen. Ein Mikroplatten-Reader kann eine im Gehäuse integrierte Magnetvorrichtung zum Abheben und Auflegen des Deckels 11 der auf einer Transportauflage aufgelegten Inkubationskassette 1 umfassen. In einem alternativen Beispiel kann der Deckel 11 mittels eines Greifnapfes oder Saugers (nicht gezeigt) abgehoben und aufgelegt werden. Der Deckel 11 ist vorzugsweise aus einem chemisch inerten Kunststoff und z.B. mittels Spritzgiessen hergestellt.

Fig. 5A,B zeigen jeweils schematische, vertikale Teilschnitte durch eine mit einem Deckel 11';11" zugedeckte Inkubationskassette 1 mit einer eingesetzten Mikroplatte 10. Die innere Wand 6 ist über einen Zwischenboden 14 mit der äusseren Wand 8 verbunden. Die äussere Wand 8, der Zwischenboden 14 und die innere Wand 6 definieren das Flüssigkeitsreservoir 9 in welches die Flüssigkeit F eingefüllt werden kann. Wie schematisch in den Figs. 5A,B gezeigt, ist der Zwischenboden 10 mit dem transparenten Abschnitt TA versehen, durch welchen Licht zur optischen Messung, z.B. einer entsprechenden Messeinrichtung (nicht gezeigt), durchlaufen kann. Das Flüssigkeitsreservoir 9 der Inkubationskassette 1 ist in den beiden gezeigten Ausführungen mit Flüssigkeit F gefüllt. Der Flüssigkeitspegel dieser Flüssigkeit F kann durch den transparenten Abschnitt TA hindurch gemessen bzw. überwacht werden.

Bei den hier gezeigten Inkubationskassetten 1 kann die innere Wand 6 abgesenkte Bereiche 15 umfassen, so dass bei aufgelegtem Deckel 11';11" jeder abgesenkte Bereich 15 die Mikroplatte 10 mit dem diese umgebenden Flüssigkeitsreservoir 9 verbindet. Es kann vorgesehen sein, dass die innere Wand 6 der Inkubationskassette 1 durchgehend weniger hoch reicht als die äussere Wand 8, so dass bei aufgelegtem Deckel 11';11" eine umlaufende Spalte die Mikroplatte 10 mit dem diese umgebenden Flüssigkeitsreservoir 9 verbindet. Damit entsteht über dem Flüssigkeitsreservoir 9 und über den Wells der Mikroplatte 10 eine zusammenhängende Gasatmosphäre.

Die Inkubationskassette 1 ist derart ausgeführt, dass Licht zur optischen Messung von unterhalb ungehindert durch den transparenten Abschnitt TA laufen kann. Mit anderen Worten sollten keine Abschnitte vorliegen, welche eine optische Achse, welche durch den jeweiligen transparenten Abschnitt TA durchläuft, nach unten blockieren könnten. Die Inkubationskassette 1 ist vorzugsweise aus einem chemisch inerten Kunststoff und z.B. mittels Spritzgiessen hergestellt. Der bzw. die transparenten Abschnitte TA können einstückig mit der Inkubationskassette 1 ausgebildet sein. Der in Fig. 5A gezeigte Deckel 11' der Inkubationskassette 1 umfasst eine im Wesentlichen flache Platte 12 und einen nach unten abstehenden und umlaufenden Rand 16', welcher vorzugsweise an diese Platte 12 angeformt ist. Durch diesen Rand 16' kann der Deckel 11' sicher und zentriert aufgesetzt und abgehoben werden, ohne dass dieser z.B. bei einer Seitwärtsverschiebung der Inkubationskassette 1 verschoben wird.

Fig. 5B zeigt ein Beispiel einer Anordnung aus Inkubationskassette 1 und aufgesetztem Deckel 11", wobei der Deckel 11" einen nach unten abstehenden und umlaufenden Rand 16" umfasst, der in eine an der Inkubationskassette 1 angeformte, umlaufende Aussparung 17 eingreift. Ferner kann am Deckel 11" ein nach unten abstehender, umlaufender Steg 18 angeformt sein, der mit einer Fläche gegen die äussere Wand 8 der Inkubationskassette 1 anschlagen kann. Somit kann der Deckel 11" weiter sicher und zentriert aufgesetzt und abgehoben werden, ohne dass dieser z.B. bei einer Seitwärtsverschiebung der Inkubationskassette 1 verschoben wird. Es wird eine zuverlässige Zentrierung des Deckels 11" auf der Inkubationskassette 1 erzielt. Zugleich wird ein Verrutschen eines aufgelegten Deckels 11" auf der Inkubationskassette 1 verhindert.

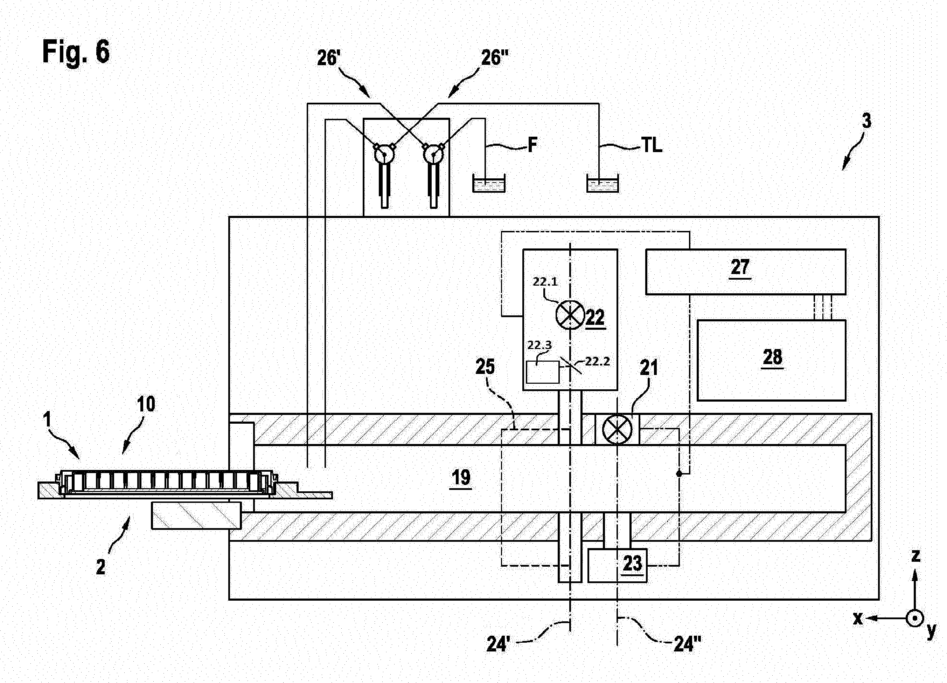

Die Fig. 6 zeigt einen Vertikalschnitt durch einen Mikroplatten-Reader 3 zum Einziehen einer Inkubationskassette 1 in einen Messraum 19, wobei eine Mikroplatte 10, z.B. eine 96-Well Standard-Mikroplatte, in die Inkubationskassette 1 eingelegt ist. Obwohl nicht gezeigt, kann die Inkubationskassette 1 mit einem Deckel abgedeckt sein. Die Mikroplatte 10 umfasst z.B. mit biologischen Strukturen enthaltende Wells. Im Zusammenhang mit der vorliegenden Erfindung umfasst der Begriff "biologische Strukturen": Gewebeteile, z.B. von Menschen, Tieren oder Pflanzen; Zellkulturen oder Teile davon; Einzelzellen; Zellorganellen; Makromoleküle wie Nukleinsäuren oder Proteine sowie Einzelmoleküle wie Nukleotide, Aminosäuren, Hormone und Metaboliten.

Der Mikroplatten-Reader 3 umfasst die Transportauflage 2 zum Aufnehmen der Inkubationskassette 1. Die Transportauflage 2 ist vorzugsweise so weit aus dem Messraum 19 des Mikroplatten-Readers 3 ausfahrbar, dass die Inkubationskassette 1 von Hand oder mittels eines Mikroplatten-Handling-Roboters (nicht dargestellt) auf die Transportauflage 2 eingelegt bzw. davon abgehoben werden kann. Die Transportauflage 2 ist hier als bereits teilweise eingefahren gezeigt, weil gerade die Mikroplatte 10 und die diese umgebende Inkubationskassette 1 in den Mikroplatten-Reader 3 eingeschoben werden. Während des Einschiebens oder Ausfahrens der Inkubationskassette 1 ist eine Klappe geöffnet, welche im geschlossenen Zustand den Messraum 19 bevorzugt lichtdicht und/oder gasdicht verschliessen kann, damit kein die Untersuchungen beeinflussendes Licht aus der Umgebung in den Messraum 19 gelangen kann und/oder die Gaskonzentration im Messraum 19 unabhängig von der Umgebung zuverlässig geregelt werden kann.

Neben dem Aufnehmen von der mit der Mikroplatte 10 bestückten Inkubationskassette 1 dient diese Transportauflage 2 auch zum Positionieren der Mikroplatte 10 mit den biologischen Strukturen (z.B. Metaboliten, Makromoleküle, Zellen oder Zellkulturen) enthaltenden Wells gegenüber Lichtquellen 21,22.1 und gegenüber Messeinrichtungen 22.3,23 des Mikroplatten-Readers 3 bzw. gegenüber den optischen Achsen 24',24" der Messeinrichtungen 22.3,23. Die Lichtquellen 21,22.1 dienen z.B. zum Herbeiführen einer Wechselwirkung zwischen zumindest einer dieser Lichtquellen 21,22.1 und biologischen Strukturen in bestimmten Wells der Mikroplatte 10, und zum Hervorrufen oder Erzeugen eines messbaren Signals. Solche Signale umfassen z.B. Fluoreszenzemission, Lumineszenzemission, reflektiertes Licht und/oder transmittiertes Licht.

Im gezeigten Ausführungsbeispiel enthält der Mikroplatten-Reader 3 ein Fluoreszenz-Modul 22 mit einer ersten Lichtquelle 22.1 inklusive einer Einrichtung zur Wellenlängenselektion, z.B. Monochromator oder Wellenlängenfilter, (nicht gezeigt) zum Anstrahlen einer Probe (Anregungslicht) entlang der ersten optischen Achse 24'. Das Fluoreszenz-Modul 22 enthält zusätzlich einen halbdurchlässigen oder dichroitischen Spiegel 22.2 zum Auskoppeln des von der Probe rückgestrahlten Lichtes (Emissionslicht) aus dem Pfad des Anregungslichtes (=erste optische Achse 24'). Über den Spiegel 22.2 wird das Emissionslicht zu einer ersten Messeinrichtung 22.3 geleitet. Im Fluoreszenz Top-Reading Modus wird eine Probe in einem Well direkt von oben aus mit dem Fluoreszenz-Modul 22 bestrahlt, und das Emissionslicht stahlt von der Probe noch oben zurück. Im Bottom-Reading Modus wird das Anregungslicht über einen Lichtleiter 25 unter die Mikroplatte geleitet und die Probe von unten durch den Boden eines jeweiligen Wells bestrahlt. Das Emissionslicht strahlt nach unten von der Probe zurück und wird über den Lichtleiter 25 zum Fluoreszenz-Modul 22 geleitet.

In dem gezeigten Ausführungsbeispiel dient die zweite Lichtquelle 21 inklusive einer Einrichtung zur Wellenlängenselektion, z.B. Monochromator oder Wellenlängenfilter, (nicht gezeigt) zum Durchstrahlen einer Probe oder biologischen Strukturen in Wells dieser Mikroplatte 10, und eine zweite Messeinrichtung 23 (hier z.B. in Form einer Photodiode) zur Messung der Absorbanz der Probe in Bezug auf die zweite optische Achse 24". Die Absorbanz wird dabei durch einen Vergleich der durch die Probe auf die zweite Messeinrichtung 23 gelangten Lichtintensität mit der transmittierten Referenz-Lichtintensität errechnet. Soll hingegen die Lumineszenz von Proben detektiert werden, kann sogar auf eine Lichtquelle verzichtet werden und das Lichtsignal mittels z.B. Photomultiplier Tubes vermessen werden.

Derartige Lichtquellen sind beispielsweise ausgewählt aus einer Gruppe, die Lichtbogenlampen, Blitzlampen, Glühlampen (wie z.B. Halogenlampen), Laser, Laserdioden und Leuchtdioden (LEDs) umfasst. Die entsprechenden Wellenlängen zum Anregen der Fluoreszenz, sowie die entsprechenden Fluorophore und deren Emissionscharakteristika, sind dem Fachmann bekannt und werden je nach Anwendung ausgewählt. Auch das nicht-invasive Durchstrahlen von Zellen oder Zellkulturen zum Erfassen der Absorption, sowie die dazu zu verwendenden Lichtquellen, sind dem Fachmann geläufig. Messeinrichtungen 22.3,23 zum Erfassen zumindest eines integralen Signals, das durch die Lichtquelle(n) 21,22.1 in oder an biologischen Strukturen in den bestimmten Wells der Mikroplatte 10 hervorgerufen oder erzeugt wurde, sind bevorzugt ausgewählt aus einer Gruppe, die Photomultiplier, Photodioden, Photodioden-Arrays und Avalanche-Dioden umfasst. Die Messeinrichtungen 22.3,23 und Lichtquellen 21,22.1, bzw. deren optischer Eingang und/oder Ausgang, sind vorzugsweise über Lichtleiter 25, wie optische Fasern oder optische Faserbündel, gekoppelt.

Die zweite Messeinrichtung 23 des Mikroplatten-Readers 3 kann zudem zur Überwachung bzw. Bestimmung des Flüssigkeitspegels im Flüssigkeitsreservoir der Mikroplatte 10 und/oder Inkubationskassette 1 verwendet werden. Hierzu kann die Inkubationskassette 1 gegenüber der zweiten optischen Achse 24" des Mikroplatten-Readers 3 derart bewegt werden, dass die zweite optische Achse 24" jeweils durch einen der transparenten Abschnitte der Inkubationskassette 1 und/oder Mikroplatte 10 laufen. Somit kann die Messeinrichtung 23 auch den Flüssigkeitspegel im Flüssigkeitskanal der Inkubationskassette 1 und/oder der Mikroplatte 10 zuverlässig messen. Bevorzugt wird die Anordnung aus Lichtquelle 21 und zweiter Messeinrichtung 23 zur Messung der Absorption der Flüssigkeit im Flüssigkeitsreservoir der Inkubationskassette 1 und/oder im Flüssigkeitsreservoir der Mikroplatte 10 verwendet.

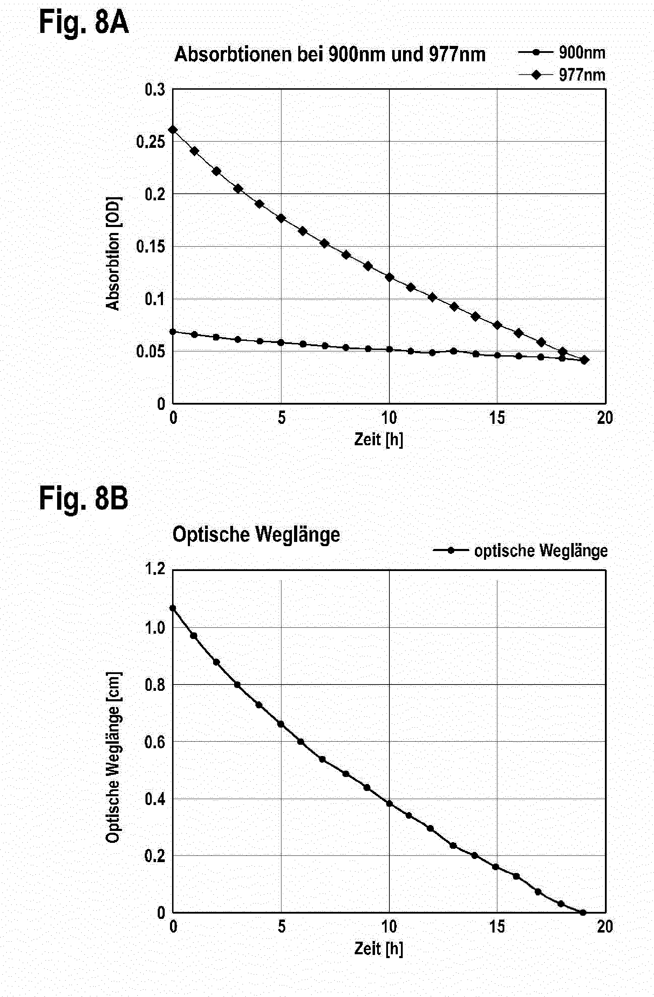

Wie zuvor beschrieben, ist die Inkubationskassette 1 mit dem transparenten Abschnitt TA versehen, durch welchen hindurch der Flüssigkeitspegel im Flüssigkeitsreservoir der Inkubationskassette 1 überwacht werden kann. Alternativ oder zusätzlich, im Falle einer transparenten Mikroplatte 10, kann der Flüssigkeitspegel im Flüssigkeitsreservoir der Mikroplatte 10 durch das transparente Material dieser Mikroplatte 10 hindurch überwacht werden. Wie bereits erwähnt, wird bevorzugt die Anordnung aus zweiter Lichtquelle 21 und zweiter Messeinrichtung 23 zur Messung der Absorption der Flüssigkeit im Flüssigkeitsreservoir der Inkubationskassette 1 und/oder Mikroplatte 10 verwendet. Weitere Details zur hier bevorzugt angewendeten Absorptionsmessung werden im Zusammenhang mit Figuren 8A,B beschrieben.

Der Mikroplatten-Reader 3 enthält z.B. zwei Injektoren 26',26", wobei über einen ersten Injektor, auch Testlösung-Injektor 26' genannt, eine Testlösung TL in die Wells der Mikroplatte abgegeben wird und über einen zweiten Injektor, auch Flüssigkeit-Injektor 26" genannt, Flüssigkeit F in das Flüssigkeitsreservoir der Inkubationskassette 1 und/oder der Mikroplatte 10 gefüllt bzw. aufgefüllt wird. Das Flüssigkeitsreservoir wird z.B. automatisiert aufgefüllt, sobald durch z.B. die Messeinrichtung 23 des Mikroplatten-Readers 3, z.B. mittels Absorptionsmessung, erfasst wird, dass der Pegel der Flüssigkeit F im Flüssigkeitsreservoir der Inkubationskassette 1 und/oder der Mikroplatte 10 einen vordefinierten Pegel unterschritten hat. Eine Steuerung 27, die z.B. zum Steuern der ersten Lichtquelle 21, der ersten

EP 3 839 481 B1

Messeinrichtung 23, der Bewegung der Transportauflage 2 des Mikroplatten-Readers 3, etc. ausgebildet sein kann, kann hierbei ferner ausgebildet sein, um den zweiten Injektor 26" zur automatisierten Abgabe der Flüssigkeit F in das Flüssigkeitsreservoir der Inkubationskassette 1 und/oder der Mikroplatte 10 zu steuern. Somit kann gewährleistet werden, dass das Flüssigkeitsreservoir der Inkubationskassette 1 und/oder der Mikroplatte 10 stets ausreichend mit Flüssigkeit F gefüllt ist, auch bei z.B. lange andauernden Analysen.

Der Mikroplatten-Reader 3 umfasst zudem einen internen bzw. integrierten Prozessor 28 oder er ist an einen externen Prozessor (nicht gezeigt) anschliessbar ausgebildet. Ein derartiger Prozessor kann somit ein in die elektronische Steuerung des Mikroplatten-Readers 3 integrierter Mikroprozessor oder ein beigestellter Personal Computer sein.

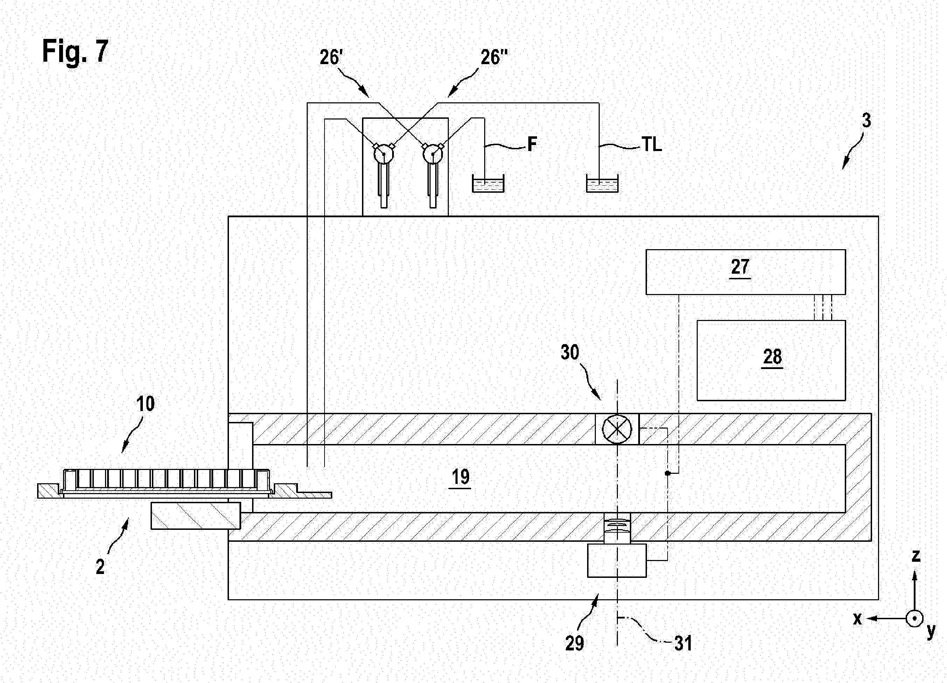

Fig. 7 zeigt einen Vertikalschnitt durch einen Mikroplatten-Reader 3 zum Einziehen von einer Mikroplatte 10 (ohne Inkubationskassette) in den Messraum 19. Die Mikroplatte 10 kann vollständig transparent sein. In der gezeigten Ausführung wird eine Anordnung aus einem Imaging-Modul mit Optik-/Linsensystem 29 und einer Beleuchtungsquelle 30 verwendet. Diese Beleuchtungsquelle 30 beleuchtet die Mikroplatte 10 von oberhalb. Das Imaging-Modul mit Optik-/Linsensystem 29 nimmt von unten durch den transparenten Boden eines Wells der Mikroplatte 10 ein Bild auf. Das Optik-/Linsensystem 29 kann dazu ausgelegt sein, Bilder optisch zu vergrössern oder zu verkleinern. Der Mikroplatten-Reader 3 kann, zusätzlich zur Beleuchtungsquelle 30 und dem Imaging-Modul mit Optik-/Linsensystem 29, Messmodule wie in Figur 6 gezeigt aufweisen. Beispielsweise kann der Flüssigkeitspegel mittels Messung der Absorption durch die Flüssigkeit bestimmt werden.

Figuren 8A,B zeigen jeweils Kurvenverläufe einer gemessenen Absorption (bei unterschiedlichen Wellenlängen) und einer bestimmten Weglänge bzw. Füllstandshöhe einer im Flüssigkeitsreservoir aufgenommenen Flüssigkeit, jeweils aufgetragen in Abhängigkeit von der Zeit. Hierbei veranschaulicht Fig. 8A die gemessene Absorption der Flüssigkeit in Abhängigkeit von der Zeit, jeweils aufgetragen bei einer Wellenlänge von 900 nm und 977 nm. Wie zu erwarten, nimmt der Flüssigkeitspegel mit fortschreitender Zeit aufgrund von Verdunstung ab.

Bei der hier bevorzugt angewendeten Absorptionsmessung kann das Beer-Lambert-Gesetz gemäss der Gleichung: $A = \varepsilon cl$ angewendet werden. Die Absorption (A) der Probe kann durch das Produkt des Extinktionskoeffizienten ($\varepsilon$) und der Konzentration (c) der Probe und der Weglänge bzw. Pfadlänge (l), durch welche die Probe gemessen wird, bestimmt werden. Bei dem Verfahren kann die Absorptionsspitze (Absorptionspeak) der Flüssigkeit, z.B. Wasser, bei Raumtemperatur (977 nm) und eine Hintergrundmessung bei 900 nm verwendet werden (siehe Messwerte, wie in Fig. 8A aufgetragen). Die Differenz der optischen Dichte (d.h. 977 nm - 900 nm) kann durch 0,18 geteilt werden, was der optischen Dichte von Wasser bei 1 cm entspricht. Das Ergebnis der obigen Berechnung ergibt die Weglänge bzw. den Flüssigkeitspegel der Flüssigkeit im Flüssigkeitsreservoir.

Unter Anwendung des Lambert-Beer-Gesetzes kann die Weglänge (d.h. Füllstandshöhe bzw. Flüssigkeitspegel) bestimmt werden, indem die Flüssigkeit (wässrige Probe) bei 900 nm gemessen wird, um eine Grundabsorptionslinie zu erhalten, und bei 977 nm gemessen wird, um die spezifische Absorption von der Flüssigkeit (wässrige Probe) zu erhalten, unter Anwendung der Gleichung:

$$Füllstandshöhe - \frac{A_{977} - A_{900}}{A_{Wasser} \, cm^{-1}}$$

wobei gilt:

$A_{977}$ -> Absorption der wässrigen Probe bei 977 nm

$A_{900}$ -> Absorption der wässrigen Probe bei 900 nm

$A_{Wasser}$ -> $A_{977}$ - $A_{900}$ Wasser in einer 1 cm Küvette

Unter Anwendung der Gleichung wird die Füllstandshöhe bzw. der Flüssigkeitspegel der Flüssigkeit im Flüssigkeitsreservoir bestimmt, wie in Fig. 8B aufgetragen. In dem gezeigten Beispiel wird ein Abfall der Füllstandshöhe von anfänglich ca. 1.1 cm auf 0 cm in annähernd 18 Stunden gemessen. Durch diese exakte Messung der Füllstandshöhe kann rechtzeitig Flüssigkeit in das Flüssigkeitsreservoir nachgefüllt werden, z.B. bei einer gemessenen Füllstandshöhe von 0.6 cm, 0.4 cm, 0.2 cm, etc.

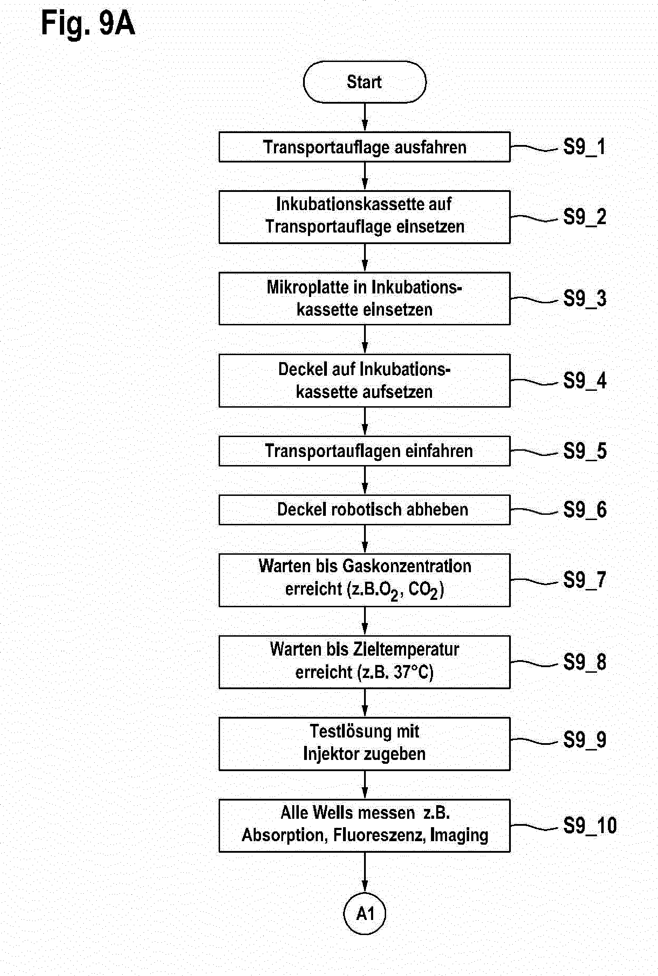

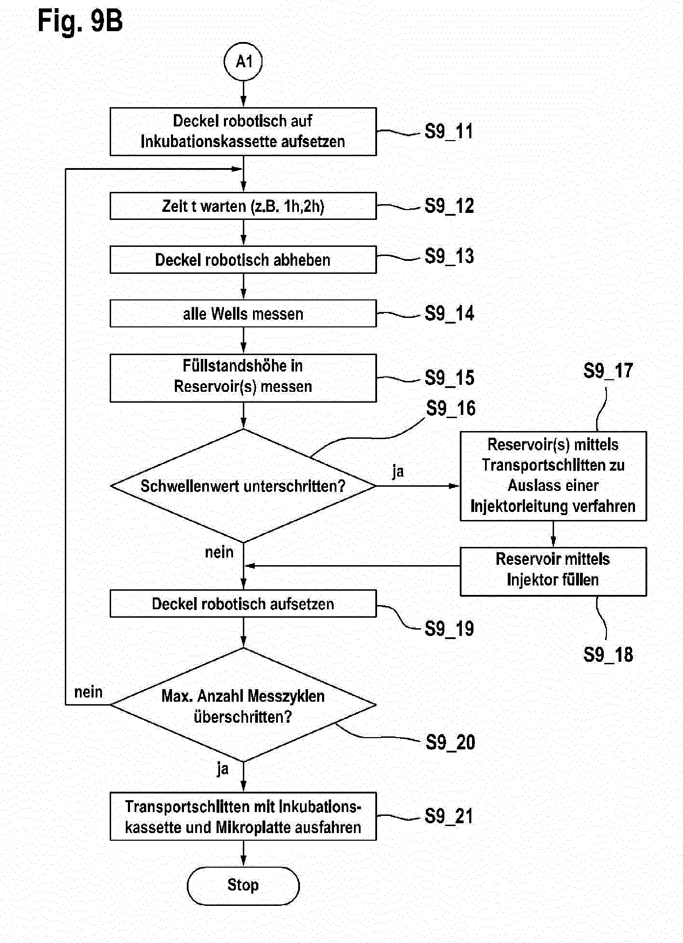

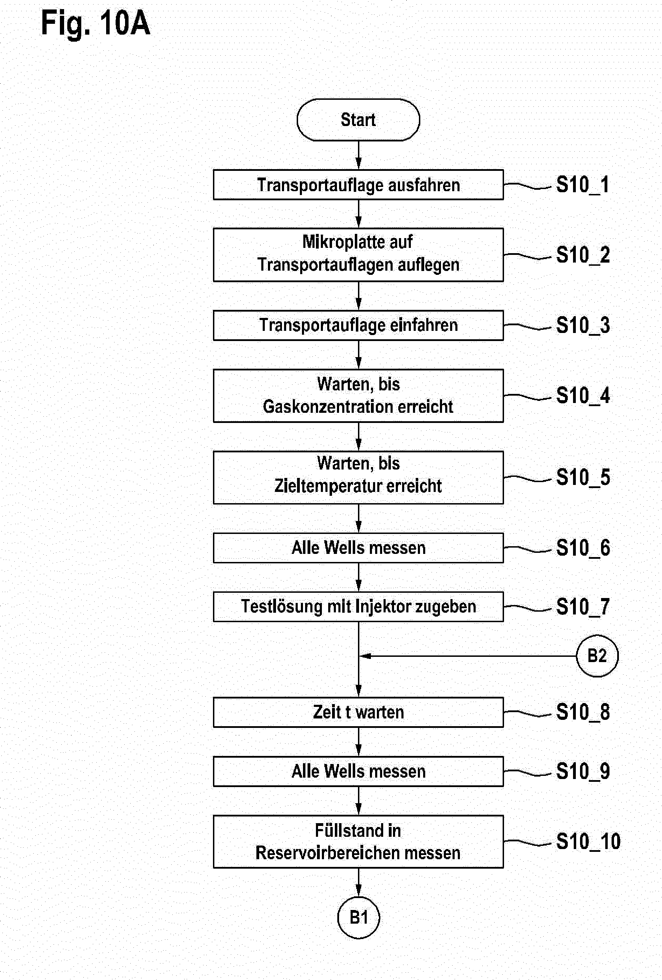

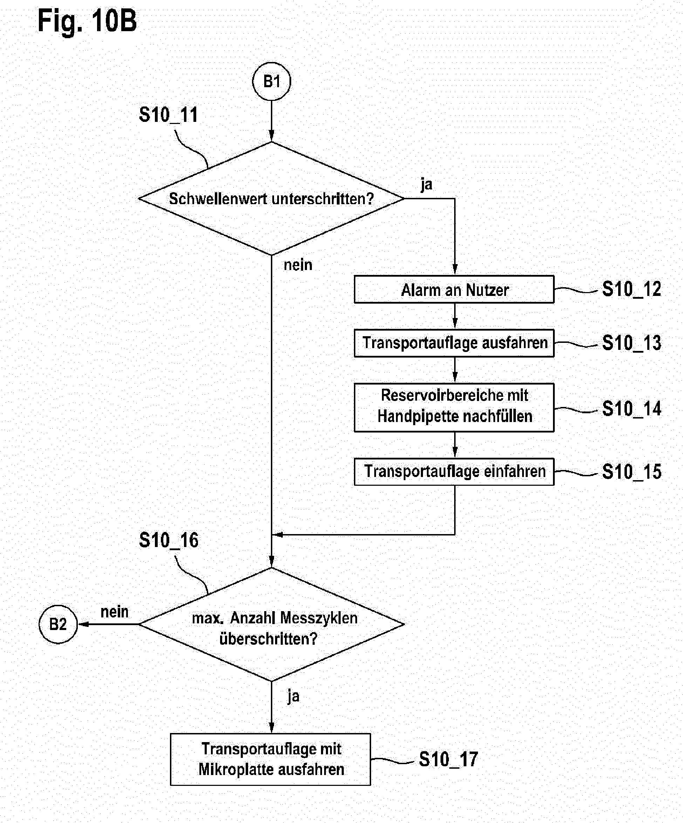

Fig. 9A,B zeigen ein Ablaufdiagramm eines Verfahrens zum Reduzieren einer Flüssigkeits-Verdunstung aus Wells einer in einer Inkubationskassette eingelegten Mikroplatte. Fig. 10A,B zeigen ein Ablaufdiagramm eines Verfahrens zum Reduzieren einer Flüssigkeits-Verdunstung aus Wells einer Mikroplatte.

Betreffend das in Fig. 9A,B gezeigte Ablaufdiagramm wird Bezug genommen auf die jeweils in Fig. 6 und 7 gezeigten Mikroplatten-Reader, in welchen eine Inkubationskassette eingeschoben wird, in der wiederum eine Mikroplatte eingelegt ist (Fig. 6) oder lediglich eine Mikroplatte (ohne Inkubationskassette) in den Mikroplatten-Reader eingeschoben wird (Fig. 7). Das Verfahren beginnt mit einem Schritt S9_1 in welchem die Transportauflage des Mikroplatten-Readers ausgefahren wird. In einem Schritt S9_2 wird die Inkubationskassette auf die Transportauflage eingesetzt. Die Inkubationskassette kann aber auch permanent eingesetzt sein. In einem Schritt S9_3 wird die Mikroplatte in die Inkubationskassette eingesetzt. Darauf wird in einem Schritt S9_4 ein Deckel auf die Inkubationskassette aufgesetzt. Die Transportauflage mit der derart vorbereiteten Inkubationskassette wird in einem Schritt S9_5 in den Mikroplatten-Reader eingefahren. Im Mikroplatten-Reader wird der Deckel robotisch abgehoben (Schritt S9_6). Im Mikroplatten-Reader wird ferner die Gaskonzentration gemessen (z.B. die Konzentration an $O_2$, $CO_2$, etc.), und in einem Schritt S9_7 wird solange abgewartet, bis eine vorbestimmte Gaskonzentration erreicht ist. Im Mikroplatten-Reader wird ferner die Temperatur gemessen, und in einem Schritt S9_8 wird solange abgewartet, bis eine vorbestimmte Zieltemperatur erreicht ist, z.B. 37°C. Sobald die vorbestimmte Gaskonzentration und/oder Zieltemperatur erreicht sind, wird in einem Schritt S9_9 die Testlösung mittels eines Injektors in vorbestimmte, mit Substanzen versehene Wells der Mikroplatte zugegeben. Allerdings kann die Testlösung auch zu einem früheren Zeitpunkt zugegeben werden, z.B. ausserhalb des Mikroplatten-Readers, d.h. noch bevor die Mikroplatte in den Mikroplatten-Reader eingeschoben wird. Ferner kann in diesem Schritt die Flüssigkeit in das Flüssigkeitsreservoir der Inkubationskassette und/oder der Mikroplatte zugegeben werden. Allerdings kann die Flüssigkeit auch zuvor zugegeben werden, z.B. ausserhalb des Mikroplatten-Readers, d.h. noch bevor die Inkubationskassette mit der Mikroplatte in den Mikroplatten-Reader eingeschoben wird.

In einem Schritt S9_10 werden die Samples in den Wells durch Messverfahren, wie z.B. Absorption, Fluoreszenz, Imaging, etc., gemessen. Anschliessend, in einem Schritt S9_11, wird der Deckel auf die Inkubationskassette aufgesetzt. Hierdurch wird die Flüssigkeits-Verdunstung aus den Wells der Mikroplatte reduziert. In einem Schritt S9_12 wird ein vorbestimmtes Zeitintervall abgewartet, z.B. 1h, 2h, etc. Nach Ablauf des vorbestimmten Zeitintervalls wird in einem Schritt S9_13 der Deckel robotisch abgehoben. In einem Schritt S9_14 werden die Samples in den jeweiligen Wells gemessen. Nachfolgend wird in einem Schritt S9_15 die Füllstandshöhe der Flüssigkeit im Flüssigkeitsreservoir der Inkubationskassette und/oder im Flüssigkeitsreservoir der Mikroplatte gemessen. In einem Schritt S9_16 wird, basierend auf dem Ergebnis der Füllstandshöhen-Messung, bestimmt, ob die Füllstandshöhe einen vorbestimmten Schwellenwert unterschritten hat oder nicht.

Wird in Schritt S9_16 bestimmt, dass die Füllstandshöhe den Schwellenwert unterschritten hat (ja), fährt das Verfahren mit einem Schritt S9_17 fort, in welchem das Flüssigkeitsreservoir der Inkubationskassette und/oder das Flüssigkeitsreservoir der Mikroplatte mittels Transportauflage zu einem Auslass einer Injektorleitung eines Injektors zum Zugeben von Flüssigkeit verfahren wird. Sobald diese Position erreicht ist, wird das entsprechende Flüssigkeitsreservoir durch den Injektor gefüllt bzw. nachgefüllt bzw. nachinjiziert (Schritt S9_18). Anschliessend fährt das Verfahren mit einem später beschriebenen Schritt S9_19 fort.

Wird in Schritt S9_16 bestimmt, dass die Füllstandshöhe den Schwellenwert nicht unterschritten hat (nein), fährt das Verfahren mit Schritt S9_19 fort, in welchem der Deckel wieder robotisch auf die Inkubationskassette aufgesetzt wird. In einem nachfolgenden Schritt S9_20 wird bestimmt, ob eine vorbestimmte maximale Anzahl von Messzyklen überschritten ist. Wird in Schritt S9_20 bestimmt, dass die maximale Anzahl von Messzyklen nicht überschritten ist (nein), kehrt das Verfahren zu Schritt S9_12 zurück. Wird in Schritt S9_20 bestimmt, dass die maximale Anzahl von Messzyklen überschritten ist (ja), fährt das Verfahren mit Schritt S9_21 fort, in welchem die Transportauflage mit der Inkubationskassette und der hierin eingelegten Mikroplatte aus dem Mikroplatten-Reader herausgefahren wird. Das Verfahren kann hiernach beendet sein.

Es ist zu erwähnen, dass in dem Ablauf die Reihenfolge der Schritte verändert werden kann. Ebenso ist das in dem Ablauf beschriebene Verfahren nicht auf die Inkubationskassette, die darin eingesetzte Mikroplatte und den auf die Inkubationskassette aufgesetzten Deckel, wie zuvor beispielhaft beschrieben, eingeschränkt zu betrachten. Neben der beschriebenen Inkubationskassette mit aufgesetztem Deckel kann auch beispielsweise eine Inkubationskassette ohne Deckel verwendet werden. In diesem Fall ist z.B. keine Robotik zum Abheben des Deckels notwendig. In der Mikroplatte kann/können in diesem Fall kein(e) Reservoir(s) vorhanden sein. Der Messraum des Mikroplatten-Readers sollte in diesem Fall möglichst klein sein. Neben dem hier beschriebenen automatisierten Nachfüllen des Reservoirs durch den Injektor kann das Nachfüllen auch manuell erfolgen.

Betreffend das in Fig. 10A,B gezeigte Ablaufdiagramm wird Bezug genommen auf die jeweils in Fig. 6 und 7 gezeigten Mikroplatten-Reader, in welchen eine Inkubationskassette eingeschoben wird, in der wiederum eine Mikroplatte eingelegt ist (Fig. 6) oder lediglich eine Mikroplatte (ohne Inkubationskassette) in den Mikroplatten-Reader eingeschoben wird (Fig. 7). Das Verfahren beginnt mit einem Schritt S10_1, in welchem die Transportauflage des Mikroplatten-Readers ausgefahren wird. In einem Schritt S10_2 wird die Mikroplatte auf die Transportauflage aufgelegt. In einem Schritt S10_3 wird die Transportauflage mit aufgelegter Mikroplatte in den Mikroplatten-Reader eingefahren. Im Mikroplatten-Reader wird die Gaskonzentration gemessen (z.B. die Konzentration an $O_2$, $CO_2$, etc.), und in einem Schritt S10_4 wird solange abgewartet, bis eine vorbestimmte Gaskonzentration erreicht ist.

Im Mikroplatten-Reader wird ferner die Temperatur gemessen, und in einem Schritt S10_5 wird solange abgewartet, bis eine vorbestimmte Zieltemperatur erreicht ist. Sobald die vorbestimmte Gaskonzentration und/oder Zieltemperatur erreicht sind, werden in einem Schritt S10_6 die Samples in den jeweiligen Wells der Mikroplatte durch Messverfahren, wie z.B. Absorption, Fluoreszenz, Imaging, etc., gemessen, um einen 'Blank' Wert für die nachfolgenden Messungen zu generieren. In einem Schritt S10_7 wird Testlösung mittels eines Injektors in vorbestimmte, mit Substanzen versehene Wells der Mikroplatte zugegeben. Allerdings kann die Testlösung auch zu einem früheren Zeitpunkt zugegeben werden, z.B. ausserhalb des Mikroplatten-Readers, d.h. noch bevor die Mikroplatte in den Mikroplatten-Reader eingeschoben wird. Ferner kann in diesem Schritt die Flüssigkeit in das Flüssigkeitsreservoir der Mikroplatte zugegeben werden. Allerdings kann die Flüssigkeit auch zuvor zugegeben werden, z.B. ausserhalb des Mikroplatten-Readers, d.h. noch bevor die Mikroplatte in den Mikroplatten-Reader eingeschoben wird. In einem Schritt S10_8 kann eine vorbestimmte Zeitdauer t abgewartet werden. In einem Schritt S10_9 können die mit der Testlösung versehenen Samples in den jeweiligen Wells gemessen werden. Nachfolgend wird in einem Schritt S10_10 die Füllstandshöhe der Flüssigkeit im Flüssigkeitsreservoir der Mikroplatte gemessen. In einem Schritt S10_11 wird, basierend auf dem Ergebnis der vorgenannten Messung, bestimmt, ob die Füllstandshöhe einen vorbestimmten Schwellenwert unterschritten hat oder nicht.

Wird in Schritt S10_11 bestimmt, dass die Füllstandshöhe den Schwellenwert unterschritten hat (ja), fährt das Verfahren mit einem Schritt S10_12 fort, in welchem ein Alarm an einen Nutzer des Mikroplatten-Readers ausgegeben werden kann. In einem Schritt S10_13 wird die Transportauflage aus dem Mikroplatten-Reader ausgefahren. Sobald die Transportauflage aus dem Mikroplatten-Reader ausgefahren ist, wird in einem Schritt S10_14 das Flüssigkeitsreservoir der Mikroplatte z.B. manuell mittels einer Handpipette mit Flüssigkeit nachgefüllt. Anschliessend wird die Transportauflage in den Mikroplatten-Reader eingefahren (Schritt S10_15). Anschliessend fährt das Verfahren mit einem später beschriebenen Schritt S10_16 fort.

Wird in Schritt S10_11 bestimmt, dass die Füllstandshöhe den Schwellenwert nicht unterschritten hat (nein), fährt das Verfahren mit Schritt S10_16 fort, in welchem bestimmt wird, ob eine vorbestimmte maximale Anzahl von Messzyklen überschritten ist.

Wird in Schritt 10_16 bestimmt, dass die maximale Anzahl von Messzyklen nicht überschritten ist (nein), kehrt das Verfahren zu Schritt S10_8 zurück. Wird in Schritt S10_16 bestimmt, dass die maximale Anzahl von Messzyklen überschritten ist (ja), fährt das Verfahren mit Schritt S10_17 fort, in welchem die Transportauflage mit der aufgelegten Mikroplatte aus dem Mikroplatten-Reader herausgefahren wird. Hiernach kann das Verfahren beendet sein.

Es ist zu erwähnen, dass die Reihenfolge der Schritte in dem beschriebenen Ablauf veränderlich ist. Ebenso ist das in dem Ablauf beschriebene Verfahren nicht auf die Mikroplatte ohne einen aufgesetzten Deckel, wie zuvor beispielhaft beschrieben, eingeschränkt zu betrachten. Neben der beschriebenen Mikroplatte ohne aufgesetzten Deckel kann auch beispielsweise eine Mikroplatte mit Deckel verwendet werden. Die Mikroplatte ist mit wenigstens einem Reservoir versehen. Im Fall der Verwendung einer Mikroplatte ohne aufgesetzten Deckel ist im Mikroplatten-Reader keine Robotik zum Abheben des Deckels notwendig. Auch hier sollte der Messraum des Mikroplatten-Readers möglichst klein bemessen sein. Das Nachfüllen des Reservoirs kann durch den Injektor oder manuell erfolgen.

Alle beschriebenen Abläufe sind als bespielhaft zu betrachten und dienen zur Erläuterung der Erfindung. Hierin beschriebene Verfahrensschritte sind nicht auf beschriebene Reihenfolgen einzuschränken. Die jeweiligen Reihenfolgen können veränderlich sein. Verfahrensschritte können, innerhalb des Schutzumfangs der Ansprüche, von beschriebenen Reihenfolgen abweichen.

Gleiche Bezugszeichen in den Figuren bezeichnen gleiche oder zumindest ähnliche Merkmale, auch wenn diese nicht in jedem Fall ausführlich beschrieben sind.

### Bezugszeichen

| | | | |
|---|---|---|---|
| 1 | Inkubationskassette | 21 | zweite Lichtquelle |
| 2 | Transportauflage | 22 | Fluoreszent-Modul |
| 3 | Mikroplatten-Reader | 22.1 | erste Lichtquelle |
| 4 | Rahmen, Inkubationsrahmen | 22.2 | halbdurchlässiger oder dichroitischer Spiegel |
| 5 | zentrale erste Öffnung | | |
| 6 | innere Wand | 22.3 | erste Messeinrichtung |
| 7 | Tragfläche von 4 | 23 | zweite Messeinrichtung |
| 8 | äussere Wand | 24' | erste optische Achse |
| 9 | Flüssigkeitsreservoir | 24" | zweite optische Achse |
| 10 | Mikroplatte | 25 | Lichtleiter |
| 11 | Deckel | 26' | erster Injektor |
| 11',11" | Deckel | 26" | zweiter Injektor |
| 12 | Platte | 27 | Steuerung |
| 13 | magnetisierbare Oberfläche | 28 | Prozessor |

(fortgesetzt)

| 14 | Zwischenboden | 29 | Imaging-Modul mit Optik-/Linsensystem |
| 15 | abgesenkter Bereich | | |
| 16' | Rand von 11' | 30 | Beleuchtungsquelle |
| 16" | Rand von 11" | 31 | optische Achse |
| 17 | Aussparung | F | Flüssigkeit |
| 18 | Steg | TA | transparenter Abschnitt |
| 19 | Messraum | TL | Testlösung |

**Patentansprüche**

1.  Verfahren zum Reduzieren einer Flüssigkeits-Verdunstung aus Wells einer Mikroplatte (10), umfassend:

    a) Bereitstellen der Mikroplatte (10) mit einer Vielzahl von Wells,
    b) Zugeben eines Samples in wenigstens eines der Wells der Mikroplatte (10),
    c) Einfahren der Mikroplatte (10) oder einer mit der Mikroplatte (10) bestückten Inkubationskassette (1) in einen Mikroplatten-Reader (3),
    d) Injizieren einer Flüssigkeit (F) in ein Flüssigkeitsreservoir (9), welches in der Mikroplatte (10) und/oder der Inkubationskassette (1) vorgesehen ist,
    e) Durchführen von Messungen auf die Samples in den jeweiligen Wells,
    f) optisches Messen eines Flüssigkeitspegels im Flüssigkeitsreservoir (9) der Mikroplatte (10) und/oder der Inkubationskassette (1) durch einen im Wesentlichen transparenten Abschnitt (TA) des Flüssigkeitsreservoirs (9) der Mikroplatte (10) und/oder des Flüssigkeitsreservoirs (9) der Inkubationskassette (1) hindurch,
    g) Nachinjizieren der Flüssigkeit (F) in das Flüssigkeitsreservoir (9) der Mikroplatte (10) und/oder in das Flüssigkeitsreservoir (9) der Inkubationskassette (1), wenn der Flüssigkeitspegel einen vorbestimmten Schwellenwert unterschreitet,
    h) Wiederholen der Schritte e) bis g), bis eine vorbestimmte Anzahl von Messzyklen erreicht ist,
    i) Ausfahren der Mikroplatte (10) oder der mit der Mikroplatte (10) bestückten Inkubationskassette (1) aus dem Mikroplatten-Reader (3),

    **dadurch gekennzeichnet,**
    **dass** das Flüssigkeitsreservoir (9) zwischen den Wells vorgesehen ist und durch eine Wandung der Mikroplatte (10) begrenzt ist und zur Aufnahme einer Flüssigkeit (F) ausgebildet ist, wobei die Mikroplatte (10) mit dem Flüssigkeitsreservoir (9) versehen ist und den wenigstens einen transparenten Abschnitt (TA) umfasst,
    und/oder
    **dass** die Inkubationskassette (1) einen Rahmen (4) zum Aufnehmen einer Mikroplatte (10) mit Wells umfasst, wobei der Rahmen (4) eine von einer inneren Wand (6) umgebene zentrale erste Öffnung (5) umfasst, deren Masse zum Einlegen einer Mikroplatte (10) ausgelegt sind, und der Rahmen (4) eine im Wesentlichen parallel zur inneren Wand (6) verlaufende äussere Wand (8) umfasst, die über einen Zwischenboden (14) an der inneren Wand (6) anschliesst, wobei das Flüssigkeitsreservoir (9) durch die beiden Wände (6,8) und den Zwischenboden (14) als ein die erste zentrale Öffnung (5) umgebendes Flüssigkeitsreservoir (9) zum Aufnehmen einer Flüssigkeit (F) gebildet ist, wobei wenigstens ein das Flüssigkeitsreservoir (9) ausbildender Abschnitt (6,8,14) der Inkubationskassette (1) wenigstens abschnittsweise mit dem mindestens einem transparenten Abschnitt (TA) versehen ist.

2.  Verfahren nach Anspruch 1, wobei die Flüssigkeit durch einen im Mikroplatten-Reader (3) umfassten Flüssigkeit-Injektor (26') oder manuell in das Flüssigkeitsreservoir (9) der Mikroplatte (10) und/oder der Inkubationskassette (1) injiziert bzw. nachinjiziert wird.

3.  Verfahren nach Anspruch 1 oder 2, wobei der Schritt c) gefolgt ist durch einen Schritt c1): Injizieren einer Testlösung zu den Samples in den Wells der Mikroplatte (10).

4.  Verfahren nach einem der vorhergehenden Ansprüche, wobei das optische Messen des Flüssigkeitspegels im Schritt f) durch optische Messung der Absorption der Flüssigkeit (F) gemessen wird.

5. Verfahren nach Anspruch 3 oder 4, wobei die Samples oder die mit der Testlösung versetzten Samples durch Messen der Absorption, Lumineszenz oder Fluoreszenz, oder durch Imaging der Samples gemessen werden.

6. Verfahren nach einem der Ansprüche 1 bis 5, wobei die Mikroplatte (10) ferner Stege (18) umfasst, welche zwischen jeweils parallel zur Wandung verlaufenden, äusseren Wells vorgesehen sind und zur Unterteilung des Flüssigkeitsreservoirs (9) in zumindest zwei Teilreservoire ausgebildet sind.

7. Mikroplatten-Reader (3) zur Durchführung des Verfahrens nach einem der Ansprüche 1 bis 6 mit einem Gehäuse und einer aus dem Gehäuse ausfahrbaren Transportauflage (2), wobei die Transportauflage (2) eine Auflagefläche zum Auflegen einer Mikroplatte (10) oder einer Inkubationskassette (1) umfasst, ferner umfassend einen Flüssigkeit- Injektor (26") zur automatisierten Abgabe von Flüssigkeit (F) in das Flüssigkeitsreservoir (9) der Mikroplatte (10) oder der Inkubationskassette (1).

8. Mikroplatten-Reader (3) nach Anspruch 7, ferner umfassend ein Fluoreszenz-Modul (22), umfassend eine erste Lichtquelle (22.1), eine Einrichtung zur Wellenlängenselektion zum Anstrahlen von Samples oder mit Testlösung versehenen Samples entlang einer ersten optischen Achse (24'), einen halbdurchlässigen oder dichroitischen Spiegel (22.2) zum Auskoppeln eines von den Samples oder mit Testlösung versehenen Samples rückgestrahlten Lichtes, und eine erste Messeinrichtung (22.3) an welche das rückgestrahlte Licht geleitet wird.

9. Mikroplatten-Reader (3) nach Anspruch 8, ferner umfassend eine zweite Messeinrichtung (23), ausgebildet zur Erfassung des Flüssigkeitspegels im Flüssigkeitsreservoir (9) der Mikroplatte (10) und/oder der Inkubationskassette (1), wobei die ausfahrbare Transportauflage (2) zum derartigen Positionieren der Mikroplatte (10) und/oder der Inkubationskassette (1) gegenüber einer zweiten optischen Achse (24") der zweiten Messeinrichtung (23) ausgebildet ist, dass die zweite optische Achse (24") durch einen transparenten Abschnitt (TA) der Mikroplatte (10) und/oder der Inkubationskassette (1) läuft.

10. Mikroplatten-Reader (3) nach Anspruch 9, wobei die zweite Messeinrichtung (23) des Mikroplatten-Readers (3) ausgebildet ist zur Absorptionsmessung der Flüssigkeit (F) im Flüssigkeitsreservoir (9) der Mikroplatte (10) und/oder der Inkubationskassette (1).

11. Mikroplatten-Reader (3) nach Anspruch 9 oder 10, ferner umfassend eine Steuerung (50), die zum Steuern der zweiten Messeinrichtung (23), einer mit der zweiten Messeinrichtung (23) zusammenwirkenden zweiten Lichtquelle (21), des Flüssigkeit-Injektors (26") zur automatisierten Abgabe von Flüssigkeit und/oder der Bewegungen der Transportauflage (2) des Mikroplatten-Readers (3) ausgebildet ist.

12. Mikroplatten-Reader (3) nach einem der Ansprüche 7 bis 11, ferner umfassend eine Vorrichtung zum Abheben und Auflegen eines Deckels (11) einer auf der Transportauflage (2) aufgelegten Inkubationskassette (1).

**Claims**

1. Method for reducing liquid evaporation from wells of a microplate (10), comprising:

   a) providing the microplate (10) with a plurality of wells,
   b) adding a sample to at least one of the wells of the microplate (10),
   c) inserting the microplate (10) or an incubation cassette (1) equipped with the microplate (10) into a microplate reader (3),
   d) injecting a liquid (F) into a liquid reservoir (9) provided in the microplate (10) and/or the incubation cassette (1),
   e) performing measurements on the samples in the respective wells,
   f) optically measuring a liquid level in the liquid reservoir (9) of the microplate (10) and/or the incubation cassette (1) through an essentially transparent section (TA) of the liquid reservoir (9) of the microplate (10) and/or the liquid reservoir (9) of the incubation cassette (1),
   g) re-injecting the liquid (F) into the liquid reservoir (9) of the microplate (10) and/or into the liquid reservoir (9) of the incubation cassette (1) when the liquid level falls below a predetermined threshold,
   h) repeating steps e) to g) until a predetermined number of measurement cycles is reached,
   i) withdrawing the microplate (10) or the incubation cassette (1) equipped with the microplate (10) from the microplate reader (3),

**characterized in**

**that** the liquid reservoir (9) is provided between the wells and is bounded by a wall of the microplate (10) and is formed to accommodate a liquid (F), wherein the microplate (10) is provided with the liquid reservoir (9) and comprises the at least one transparent section (TA), and/or

**that** the incubation cassette (1) comprises a frame (4) for receiving a microplate (10) with wells, wherein the frame (4) comprises a central first opening (5) surrounded by an inner wall (6), the dimensions of which are designed for inserting a microplate (10), and wherein the frame (4) comprises an outer wall (8) extending substantially parallel to the inner wall (6) and which adjoins the inner wall (6) via an intermediate bottom (14), wherein the liquid reservoir (9) is formed by the two walls (6, 8) and the intermediate bottom (14) as a liquid reservoir (9) surrounding the first central opening (5) for receiving a liquid (F), wherein at least one section (6,8,14) of the incubation cassette (1) forming the liquid reservoir (9) is provided at least sectionally with the at least one transparent section (TA).

2. Method according to claim 1, wherein the liquid is injected or re-injected into the liquid reservoir (9) of the microplate (10) and/or the incubation cassette (1) by a liquid injector (26') comprised in the microplate reader (3) or manually.

3. Method of claim 1 or 2, wherein step c) is followed by a step c1): injecting a test solution into the samples in the wells of the microplate (10).

4. Method according to any one of the preceding claims, wherein optically measuring the liquid level in step f) is performed by optically measuring the absorption of the liquid (F).

5. Method according to claim 3 or 4, wherein the samples or the samples mixed with the test solution are measured by measuring the absorption, luminescence or fluorescence, or by imaging the samples.

6. Method according to any one of claims 1 to 5, wherein the microplate (10) further comprises webs (18) which are provided between outer wells running parallel to the wall and are designed to subdivide the liquid reservoir (9) into at least two partial reservoirs.

7. Microplate reader (3) for carrying out the method according to any one of claims 1 to 6 with a housing and a transport support (2) extendable from the housing, wherein the transport support (2) comprises a support surface for placing a microplate (10) or an incubation cassette (1), further comprising a liquid injector (26") for automated delivery of liquid (F) into the liquid reservoir (9) of the microplate (10) or the incubation cassette (1).

8. Microplate reader (3) according to claim 7, further comprising a fluorescence module (22), comprising a first light source (22.1), a means for wavelength selection for illuminating irradiating samples or samples provided with a test solution along a first optical axis (24'), a semitransparent or dichroic mirror (22.2) for outcoupling of light reflected by the samples or by the samples provided with a test solution, and a first measuring device (22.3) to which the reflected light is conducted.

9. Microplate reader (3) according to claim 8, further comprising a second measuring device (23), designed for recording the liquid level in the liquid reservoir (9) of the microplate (10) and/or the incubation cassette (1), wherein the extendable transport support (2) is designed for positioning the microplate (10) and/or the incubation cassette (1) in relation to a second optical axis (24") of the second measuring device (23) such that the second optical axis (24") passes through a transparent section (TA) of the microplate (10) and/or the incubation cassette (1).

10. Microplate reader (3) according to claim 9, wherein the second measuring device (23) of the microplate reader (3) is designed for the absorption measurement of the liquid (F) in the liquid reservoir (9) of the microplate (10) and/or the incubation cassette (1).

11. Microplate reader (3) according to claim 9 or 10, further comprising a controller (50) designed for controlling the second measuring device (23), a second light source (21) interacting with the second measuring device (23), the liquid injector (26") for the automated dispensing of liquid and/or the movements of the transport support (2) of the microplate reader (3).

12. Microplate reader (3) according to any one of claims 7 to 11, further comprising a device for lifting off and placing a lid (11) on an incubation cassette (1) placed on the transport support (2).

**Revendications**

1. Procédé de réduction d'une vaporisation de fluide depuis les puits d'une plaque de microtitration (10), comprenant les étapes suivantes :

   a) mise à disposition de la plaque de microtitration (10) comprenant une pluralité de puits,
   b) ajout d'un échantillon dans au moins un des puits de la plaque de microtitration (10),
   c) introduction de la plaque de microtitration (10) ou d'une cassette d'incubation (1) équipée de la plaque de microtitration (10) dans un lecteur de plaque (3),
   d) injection d' un fluide (F) dans un réservoir de fluide (9) qui est prévu dans la plaque de microtitration (10) et/ou dans la cassette d'incubation (1),
   e) réalisation de mesures sur les échantillons de chacun des puits respectifs,
   f) mesure optique d'un niveau de liquide dans le réservoir de fluide (9) de la plaque de microtitration (10) et/ou de la cassette d'incubation (1) par une section (TA), essentiellement transparente, du réservoir de fluide (9) de la plaque de microtitration (10) et/ou du réservoir de fluide (9) de la cassette d'incubation (1),
   g) réinjection de fluide (F) dans le réservoir de fluide (9) de la plaque de microtitration (10) et/ou dans le réservoir de fluide (9) de la cassette d'incubation (1) lorsque le niveau de fluide passe sous une valeur seuil prédéterminée,
   h) répétition des étapes e) à g) jusqu'à ce qu'un nombre prédéterminé de cycles de mesure soit atteint,
   i) retrait de la plaque de microtitration (10) ou de la cassette d'incubation (1) équipée de la plaque de microtitration (10) du lecteur de plaque (3),

   **caractérisé**

   **en ce que** le réservoir de fluide (9) est prévu entre les puits et est délimité par une paroi de la plaque de microtitration (10) et est conçu pour recevoir un fluide (F), la plaque de microtitration (10) étant équipée du réservoir de fluide (9) et comportant la section (TA) transparente, au moins au nombre de une, et/ou
   **en ce que** la cassette d'incubation (1) comprend un cadre (4) de réception d'une plaque de microtitration (10) comprenant des puits, le cadre (4) présentant une première ouverture centrale (5) entourée d'une paroi intérieure (6), dont la matière est conçue pour insérer une plaque de microtitration (10), et le cadre (4) comprenant une paroi extérieure (8) qui s'étend essentiellement parallèlement à la paroi intérieure (6) et qui se raccorde à la paroi intérieure (6) par un corps intermédiaire (14), le réservoir de fluide (9) étant formé par les deux parois (6, 8) et le corps intermédiaire (14) en tant que réservoir de fluide (9), entourant la première ouverture centrale (5), destiné à recevoir un fluide (F), au moins une section (6, 8, 14) de la cassette d'incubation, formant le réservoir (9), étant munie, au moins par sections, de la section (TA) transparente, au moins au nombre de une.

2. Procédé selon la revendication 1, dans lequel le fluide est injecté ou réinjecté dans le réservoir de fluide (9) de la plaque de microtitration (10) et/ou de la cassette d'incubation (1) par un injecteur de fluide (26') intégré dans le lecteur de plaque (3) ou manuellement.

3. Procédé selon la revendication 1 ou 2, dans lequel l'étape c) est suivie d'une étape c1) : injection d'une solution de test dans les échantillons des puits de la plaque de microtitration (10).

4. Procédé selon l'une des revendications précédentes, dans lequel la mesure optique du niveau de fluide à l'étape f) se fait par mesure optique de l'absorption du fluide (F).

5. Procédé selon la revendication 3 ou 4, dans lequel les échantillons ou les échantillons mélangés avec la solution de test sont mesurés par mesure de l'absorption, de la luminescence ou de la fluorescence, ou par imagerie des échantillons.

6. Procédé selon l'une des revendications 1 à 5, dans lequel la plaque de microtitration (10) comprend de plus des entretoises (18) qui sont prévues entre les puits extérieurs respectifs s'étendant parallèlement à la paroi et sont conçus pour subdiviser le réservoir de fluide (9) en au moins deux réservoirs partiels.

7. Lecteur de plaque (3) destiné à réaliser le procédé selon l'une des revendications 1 à 6, comprenant un boîtier et une embase de transport (2) pouvant être extraite du boîtier, l'embase de transport (2) comprenant une surface de pose destinée à placer une plaque de microtitration (10) ou une cassette d'incubation (1), comprenant en outre un injecteur de fluide (26") permettant de déposer de façon automatisée du fluide (F) dans le réservoir de fluide (9) de la plaque de microtitration (10) ou de la cassette d'incubation (1).

**8.** Lecteur de plaque (3) selon la revendication 7, comprenant en outre un module de fluorescence (22), qui comporte une première source de lumière (22.1), un dispositif de sélection de longueur d'onde destiné à illuminer des échantillons ou des échantillons munis de la solution de test le long d'un premier axe optique (24'), un miroir (22.2) semi-réfléchissant ou dichroïque permettant de découpler une lumière renvoyée par les échantillons ou les échantillons munis de la solution de test, et un premier dispositif de mesure (22.3), auquel est transmise la lumière renvoyée.

**9.** Lecteur de plaque (3) selon la revendication 8, comprenant outre un second dispositif de mesure (23) conçu pour enregistrer le niveau de fluide dans le réservoir de fluide (9) de la plaque de microtitration (10) et/ou de la cassette d'incubation (1), l'embase de transport extractible (2) étant conçue pour positionner la plaque de microtitration (10) et/ou la cassette d'incubation (1) par rapport à un second axe optique (24") du second dispositif de mesure (23) de façon telle que le second axe optique (24") s'étend à travers une section transparente (TA) de la plaque de microtitration (10) et/ou de la cassette d'incubation (1).

**10.** Lecteur de plaque (3) selon la revendication 9, dans lequel le second dispositif de mesure (23) du lecteur de plaque (3) est conçu pour mesurer l'absorption du fluide (F) dans le réservoir de fluide (9) de la plaque de microtitration (10) et/ou de la cassette d'incubation (1).

**11.** Lecteur de plaque (3) selon la revendication 9 ou 10, comprenant en outre un dispositif de commande (50) qui est conçu pour commander le second dispositif de mesure (23), une seconde source de lumière (21) coopérant avec le second dispositif de mesure (23), l'injecteur de fluide (26") permettant de déposer de façon automatisée du fluide, et/ou pour commander les déplacements de l'embase de transport (2) du lecteur de plaque (3).

**12.** Lecteur de plaque (3) selon l'une des revendications 7 à 11, comprenant en outre un dispositif permettant de retirer et de déposer un couvercle (11) d'une cassette d'incubation (1) placée sur l'embase de transport (2).

Fig. 1

## Fig. 2A

## Fig. 2B

## Fig. 3A

## Fig. 3B

Fig. 4

# Fig. 5A

# Fig. 5B

# Fig. 6

Fig. 7

# Fig. 8A

### Absorbtionen bei 900nm und 977nm

● 900nm
◆ 977nm

Absorbtion [OD] / Zeit [h]

# Fig. 8B

### Optische Weglänge

● optische Weglänge

Optische Weglänge [cm] / Zeit [h]

# Fig. 9A

```
                    ( Start )
                        │
                        ▼
        ┌──────────────────────────────┐
        │  Transportauflage ausfahren  │────  S9_1
        └──────────────────────────────┘
                        │
                        ▼
        ┌──────────────────────────────┐
        │  Inkubationskassette auf     │────  S9_2
        │  Transportauflage einsetzen  │
        └──────────────────────────────┘
                        │
                        ▼
        ┌──────────────────────────────┐
        │  Mikroplatte in Inkubations- │────  S9_3
        │  kassette einsetzen          │
        └──────────────────────────────┘
                        │
                        ▼
        ┌──────────────────────────────┐
        │  Deckel auf Inkubations-     │────  S9_4
        │  kassette aufsetzen          │
        └──────────────────────────────┘
                        │
                        ▼
        ┌──────────────────────────────┐
        │  Transportauflagen einfahren │────  S9_5
        └──────────────────────────────┘
                        │
                        ▼
        ┌──────────────────────────────┐
        │  Deckel robotisch abheben    │────  S9_6
        └──────────────────────────────┘
                        │
                        ▼
        ┌──────────────────────────────┐
        │  Warten bis Gaskonzentration │────  S9_7
        │  erreicht (z.B. $O_2$, $CO_2$) │
        └──────────────────────────────┘
                        │
                        ▼
        ┌──────────────────────────────┐
        │  Warten bis Zieltemperatur   │────  S9_8
        │  erreicht (z.B. 37°C)        │
        └──────────────────────────────┘
                        │
                        ▼
        ┌──────────────────────────────┐
        │  Testlösung mit              │────  S9_9
        │  Injektor zugeben            │
        └──────────────────────────────┘
                        │
                        ▼
        ┌──────────────────────────────┐
        │  Alle Wells messen  z.B.     │────  S9_10
        │  Absorption, Fluoreszenz,    │
        │  Imaging                     │
        └──────────────────────────────┘
                        │
                        ▼
                    ( A1 )
```

# Fig. 9B

A1

Deckel robotisch auf
Inkubationskassette aufsetzen — S9_11

Zeit t warten (z.B. 1h,2h) — S9_12

Deckel robotisch abheben — S9_13

alle Wells messen — S9_14

Füllstandshöhe in
Reservoir(s) messen — S9_15

S9_16

Schwellenwert unterschritten? — ja → Reservoir(s) mittels
Transportschlitten zu
Auslass einer
Injektorleitung verfahren — S9_17

nein

Reservoir mittels
Injektor füllen — S9_18

Deckel robotisch aufsetzen — S9_19

nein — Max. Anzahl Messzyklen
überschritten? — S9_20

ja

Transportschlitten mit Inkubationskassette und Mikroplatte ausfahren — S9_21

Stop

# Fig. 10A

```
                    ┌─────────────┐
                    │    Start    │
                    └─────────────┘
                           │
                           ▼
        ┌──────────────────────────────────┐
        │   Transportauflage ausfahren     │────── S10_1
        └──────────────────────────────────┘
                           │
                           ▼
        ┌──────────────────────────────────┐
        │         Mikroplatte auf          │
        │   Transportauflagen auflegen     │────── S10_2
        └──────────────────────────────────┘
                           │
                           ▼
        ┌──────────────────────────────────┐
        │    Transportauflage einfahren    │────── S10_3
        └──────────────────────────────────┘
                           │
                           ▼
        ┌──────────────────────────────────┐
        │           Warten, bis            │
        │    Gaskonzentration erreicht     │────── S10_4
        └──────────────────────────────────┘
                           │
                           ▼
        ┌──────────────────────────────────┐
        │           Warten, bis            │
        │     Zieltemperatur erreicht      │────── S10_5
        └──────────────────────────────────┘
                           │
                           ▼
        ┌──────────────────────────────────┐
        │         Alle Wells messen        │────── S10_6
        └──────────────────────────────────┘
                           │
                           ▼
        ┌──────────────────────────────────┐
        │  Testlösung mit Injektor zugeben │────── S10_7
        └──────────────────────────────────┘
                           │◄──────────────────── ( B2 )
                           ▼
        ┌──────────────────────────────────┐
        │          Zeit t warten           │────── S10_8
        └──────────────────────────────────┘
                           │
                           ▼
        ┌──────────────────────────────────┐
        │         Alle Wells messen        │────── S10_9
        └──────────────────────────────────┘
                           │
                           ▼
        ┌──────────────────────────────────┐
        │           Füllstand in           │
        │    Reservoirbereichen messen     │────── S10_10
        └──────────────────────────────────┘
                           │
                           ▼
                        ( B1 )
```

# Fig. 10B

B1

S10_11

Schwellenwert unterschritten? — ja

nein

Alarm an Nutzer — S10_12

Transportauflage ausfahren — S10_13

Reservoirbereiche mit Handpipette nachfüllen — S10_14

Transportauflage einfahren — S10_15

S10_16

B2 ← nein — max. Anzahl Messzyklen überschritten?

ja

Transportauflage mit Mikroplatte ausfahren — S10_17

**EP 3 839 481 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2943797 B1

- US 4800164 A